# EUROPEAN PATENT APPLICATION

(11) **EP 4 672 255 A2**
(43) Date of publication of application: **31.12.2025**
(21) Application number: 25208378.7
(22) Date of filing: 08.10.2022
(51) Int. Cl.: G16H 20/40

(54) **METHOD AND SYSTEM FOR DOSE QUANTIFICATION**

(30) Priority: 09.10.2021 AU 2021903247
(62) Divisional of application: 22877717.3
(71) Applicant: Australian Nuclear Science And Technology Organisation, Lucas Heights, New South Wales 2234 (AU)
(72) Inventor: SAFAVI-NAEINI, Mitra, Lucas Heights, 2234 (AU); CHACON, Andrew Stephen, Lucas Heights, 2234 (AU)
(74) Representative: Fleuchaus & Gallo Partnerschaft mbB

(57) **Abstract**

A radiation dose quantification method and system, the method comprising: detecting, with one or more detectors with respective sensitive volumes, gamma-rays emitted as a result of capture of neutrons by a composition in a subject subjected to an irradiation program, the composition comprising one or more thermal neutron capture agents, the neutrons having been generated by non-elastic collisions between a primary beam of particles and nuclei in the subject, wherein the particles consist of any one or more of protons, deuterons, tritons and heavy ions, the irradiation program comprises at least one period of irradiation with a beam duration that includes beam-on periods and beam-off periods; applying at least one predefined energy window or filter configured to accept only detection events in the one or more detectors resulting from gamma-rays with an energy indicative of selected gamma-rays arising from the capture of thermal neutrons by the one or more thermal neutron capture agents, wherein the thermal neutrons are neutrons with energies below approximately 0.4 eV; applying a timing window configured to reject or ignore detection events in the one or more detectors resulting from at least prompt gamma-rays produced in non-neutron capture events; and determining the radiation dose of neutron radiation received by the subject during the irradiation program from at least the accepted detection events or determining a dose map of the radiation received by the subject from at least the accepted detection events.

## Description

### Related Application

This application is based on and claims the benefit of the filing and priority dates of AU application no. 2021903247 filed 9 October 2021.

### Field of the Invention

The present invention relates to a method and a system for radiation dose quantification, of particular application in quantifying neutron capture events in particle therapy.

### Background of the Invention

WO 2019/051557 A1 discloses an irradiation method and composition of use in particular in what is termed 'Neutron Capture Enhanced Particle Therapy' (or NCEPT). Thermal neutrons are generated within a patient by beam-target nuclear interactions in and around the treatment site, and the effectiveness of the therapy is boosted by capturing the thermal neutrons using neutron capture agents such as tumour-specific ¹⁰B- or ¹⁵⁷Gd-based agents. This neutron capture releases high-LET (linear energy transfer) secondary particles and, with ¹⁰B- or ¹⁵⁷Gd-based agents, gamma-rays with energies of 478 keV (¹⁰B) or 79.5 keV, 182 keV, 6.75 MeV, 7.86 MeV and 7.94 MeV (¹⁵⁷Gd), respectively.

Verburg et al., in Energy- and time-resolved detection of prompt gamma-rays for proton range verification (Phys. Med. Biol. 58(20) (2013) L37-L49) disclose a a prompt gamma-ray detector for proton beam range verification, with acquisition synchronized to cyclotron radio frequency to separate prompt gamma-ray signals from later-arriving neutron-induced background, energy resolution and background-event reduction.

Melek Zarifi discloses, in Prompt gamma-ray imaging for on-line tracking of the bragg peak in proton radiation therapy (University of Wollongong Research Online, 26 Nov 2015, XP055347253), strategies for detection of prompt gamma-rays emitted in water and PMMA phantoms from high energy proton beam irradiations.

Fondazione TERA, in Sistema Aqua "Advanced Quality Assurance" Per II Centro Nazionale Di Adroterapia Oncologica (15 Feb 2008, XP055005634), discloses a beam-based PET system for session-by-session control of dose localization delivered by a carbon ion beam, including nuclear scattering tomography using a 1000 MeV synchrotron produced proton beam.

Hirano et al., in Monte Carlo simulation of small OpenPET prototype with 11C beam irradiation: effects of secondary particles on in-beam imaging (Phys. Med. Biol. 59 (2014) 1623-1640), disclose an evaluation of the effects of secondary particles on in-beam PET imaging using Monte Carlo simulation of a small OpenPET prototype in which a PMMA phantom is irradiated with a ¹¹C beam.

U.S. Patent Publication No. 2016/0187270 A1 discloses a system for detecting and identifying nuclear materials (e.g. highly enriched uranium) that relies on the emission of delayed neutrons present in the decay of fission products and time-of-flight measurement in the first 1 µs after the inducing radiation pulse, and pulse height data analysis for neutrons and gamma-rays. However, planning, optimization and delivery of NCEPT is more complex than for previous particle therapies, as the internally-generated thermal neutron fluence depends on factors such as treatment volume size and depth, and the cellular concentration of the neutron capture agent.

### Summary of the Invention

It is an object of the present invention to facilitate radiation dose quantification in NCEPT.

According to a first broad aspect, the invention provides a radiation dose quantification method, comprising:
detecting, with one or more detectors with respective sensitive volumes, gamma-rays emitted as a result of capture of neutrons by a composition in a subject subjected to an irradiation program, the composition comprising one or more thermal neutron capture agents, the neutrons having been generated by non-elastic collisions between a primary beam of charged particles and nuclei in the subject, wherein the charged particles consist of any one or more of protons, deuterons, tritons and heavy ions, the irradiation program comprises at least one period of irradiation with a beam duration that includes beam-on periods and beam-off periods;
applying at least one predefined energy window or filter configured to accept only detection events in the one or more detectors resulting from gamma-rays with an energy indicative of selected (e.g. principal or sole) gamma-rays arising from the capture of thermal neutrons by the one or more thermal neutron capture agents, wherein the thermal neutrons are neutrons with energies below approximately 0.4 eV;
applying a timing window configured to reject or ignore detection events in the one or more detectors resulting from at least prompt gamma-rays produced in non-neutron capture events; and
determining the dose of thermal neutron radiation received by the subject during the irradiation program from at least the accepted detection events (viz. the counted gamma-rays) or determining a dose map of the thermal neutron radiation received by the subject from at least the accepted detection events.

Determining the radiation dose or determining the dose map may additionally employ beam data indicative of primary beam position (i.e. within the subject) and primary beam energy corresponding to the respective accepted detection events.

It should be noted that applying the at least one predefined energy window can be performed online (i.e., as the detection events are being collected) or offline (i.e., after data collection, by filtering the detection events). However, events falling outside the energy window are unlikely to be of value for present purposes. Likewise, applying the timing window can be performed online or offline. Thus, in various embodiments, one or both of applying the energy window and applying the timing window are performed online, and in other embodiments one or both of applying the energy window and applying the timing window are performed offline.

The one or more detectors may be sensitive to the angle of arrival of gamma-rays, which is especially advantageous in determining a dose map.

The method may include detecting the neutron capture gamma-rays, subject to the timing window and the energy window, for a period of at least approximately 10^{5.8} ns, or for a period of 10^{5.8} ns to 2 × 10⁶ ns, after the end of the respective beam-on periods.

In another embodiment, the method includes detecting all of the neutron capture gamma-rays that fall within a pre-determined energy window of interest for offline processing (such as by subsequently, applying the timing window offline).The charged particles be ionized H (i.e., protons), ⁴He (i.e. alpha particles, which are generally regarded as heavy ions), C, O and/or Si, including in particular ⁹C, ¹⁰C, ¹¹C, ¹²C, ¹⁵O, ¹⁵O and high n isotopes of Si (which are generally regarded as heavy ions).

The composition is advantageously preferentially absorbed by a malignant (e.g. cancerous) target tissue.

The primary beam typically obtains appropriate energy by cyclotron or synchrotron acceleration of the particles.

The method may apply a plurality of energy windows if the neutron capture agent or agents produce gamma-rays of more than one energy. In addition, in embodiments that employ more than one detector, different energy windows may be defined for different detectors, according to the gamma-rays the respective detector is configured or intended to detect.

The method may include configuring the timing window to reject the prompt gamma-rays by rejecting gamma-rays that arrive in the respective sensitive volumes from the start of each beam-on period to approximately 11 ns, approximately 12 ns, or from 10 to 12 ns, after the end of the respective beam-on period.

The method may include configuring the timing window also to reject detection events in the respective sensitive volumes attributable, based on timing, to fast neutrons.

This may include configuring the timing window to reject detection events from the start of each beam-on period to at least 30 ns, at least 40 ns , at least 49 ns, at least 50 ns, or 30 to 50 ns, after the end of the respective beam-on period.

The method may include configuring the timing window also to reject detection events in the respective sensitive volumes attributable, based on timing, to neutrons with energies between 0.4 eV and 1 MeV.

This may include configuring the timing window to reject detection events from the start of each beam-on period to between 50 ns and 10⁴ ns after the end of the respective beam-on period.

The method may include configuring the timing window to reject detection events in a detection period that has a ratio of true positive detection events to false positive detection events, ascertained empirically or by simulation, to be less than 1.4 or less than 1.5, wherein a true positive detection event is an event that satisfies the timing window and the energy window and arises from a neutron capture event, and a false positive detection event is an event that satisfies the timing window and the energy window and arises from other than a neutron capture event.

Thus, a ratio of greater than 1.5 is workable, but higher ratio values produce a better quality image or more accurate validation of the delivered dose at one or more specific positions in the subject/target.

The method may include at least partially shielding the one or more detectors (and in particular the scintillators thereof) with single or multiple (e.g. four) layers of neutron absorbing and/or scattering material, wherein the material is different from the one or more neutron capture agents. In some embodiments, the method desirably includes shielding the one or more detectors on all sides except where the one or more detectors face the subject (or the relevant volume thereof).

Neutron absorption (whereby the shielding acts as a passive filter) is more practical with thermal neutrons, while scattering/deflecting is generally the more practical approach with fast neutrons in order to scatter a substantial fraction of fast neutrons away from the detector.

In such embodiments, the shielding may also be disposed over the front face of the one or more detectors so as to absorb and/or scatter thermal neutrons from the one or more thermal neutron capture agents. If the neutron capture agent is or includes a ¹⁰B-based agent, any trace boron present as a dopant in components of the dose quantification system (such as silicon substrates or PCBs) may produce a background that compromises the measurement of the gamma-rays arising from the capture of thermal neutrons by the thermal neutron capture agent; this makes some shielding effectively essential in any practical implementation that employs a ¹⁰B-based agent.

The shielding may optionally also be disposed over the side or sides of the one or more detectors for the same reason. If the neutron capture agent is or includes a ¹⁰B-based agent, and trace boron is present (or is present in excessive amounts), at least some shielding of the side(s) may also be effectively essential in any practical implementation.

The neutron absorbing or scattering material(s) are selected based on their neutron absorption and scattering cross-section and thick enough to block and scatter most of the neutrons away from the detector, but thin enough not to absorb or scatter too high a fraction of the gamma-rays generated due to neutron capture. For example, the material may comprise a high density plastic (such as high density polyethylene), paraffin, cadmium, gadolinium, boron, boron-carbide, lead, brass and/or hafnium.

In some examples, the one or more neutron capture agents are ¹⁰B-based and/or ¹⁵⁷Gd-based. For example, the one or more neutron capture agents may be ¹⁰B-based such that the gamma-rays have an energy of 478 keV, and/or ¹⁵⁷Gd-based such that the gamma-rays have an energy of 79.5 keV, 182 keV, 6.75 MeV, 7.86 MeV and/or 7.94 MeV.

The method may comprise planning a radiation therapy involving generation of thermal neutrons within a patient by beam-target nuclear interactions in and around the treatment site.

In some examples, the neutron capture agent is ¹⁰B-based and the one or more detectors comprise any one or more of CdTe, CZT, LYSO:Ce and LaBr₃:Ce detectors. In these examples, the beam duration may be of from 1 to 10 *µ*s, or of approximately 1 *µ*s. The method may comprise shielding the one or more detectors with a thermal neutron absorbing material comprising natural Cd, natural Gd, Pb, brass, a high density plastic and/or natural Hf.

In other examples, the neutron capture agent is ¹⁵⁷Gd-based and the one or more detectors comprise any one or more of LSO:Ce, BGO and PbWO₄ detectors. In these examples, the beam duration may be of from 10 *µ*s to 100 ms. If the primary beam is a carbon ion beam, the beam duration may be approximately 1 ms; if the primary beam is a helium ion beam, the beam duration may be approximately 10 *µ*s. The method may include shielding the one or more detectors with a thermal neutron absorbing material comprising natural B and/or natural Hf.

The method (and system, see below) may employ any combination of the following:

| **Neutron capture Agent** | **Detector** | **Shielding Material** | **Beam Duration** | **Primary Beam** |
|---|---|---|---|---|
| ¹⁵⁷Gd | LSO:Ce | natural B | 1 ms | carbon ion |
| | BGO | natural Hf | 1 *µ*s | |
| | PbWO₄ | none | 10 ms | |
| | | | 100 ms | |
| | | | 1 *µ*s-100 ms | |

| **Neutron Capture Agent** | **Detector** | **Shielding Material** | **Beam Duration** | **Primary Beam** |
|---|---|---|---|---|
| ¹⁰B | CdTe | None | 1 *µ*s | carbon ion |
| | CZT | natural Cd | 10 *µ*s | |
| | LaBr₃:Ce | natural Gd | 1-10 *µ*s | |
| | | natural Hf | | |

| **Neutron Capture Agent** | **Detector** | **Shielding Material** | **Beam Duration** | **Primary Beam** |
|---|---|---|---|---|
| ¹⁵⁷Gd | LSO:Ce | natural B | 10 *µ*s | helium ion |
| | BGO | natural Hf | 1 ms | |
| | PbWO₄ | none | 10 ms | |
| | | | 100 ms | |
| | | | 10 *µ*s-100 ms | |

| **Neutron Capture Agent** | **Detector** | **Shielding Material** | **Beam Duration** | **Primary Beam** |
|---|---|---|---|---|
| ¹⁰B | CdTe | none | 1 *µ*s | helium ion |
| | CZT | natural Cd | 10 *µ*s | |
| | LaBr₃:Ce | natural Gd | 1-10 *µ*s | |
| | | natural Hf | | |

Pb, brass and Cd may also be used to shield the sides of the detector(s) from fast neutrons without excessively thermalizing the neutrons.

Such combinations, in particular examples, may be employed was timing masks that provide a ratio of true positives to false positives *R_{TF}* (as defined herein) of greater than 1.4 or, advantageously, greater than 1.5.

According to a second broad aspect, the invention provides a radiation dose quantification system, comprising:
one or more detectors with respective sensitive volumes, and configured to detect gamma-rays emitted as a result of capture of neutrons by a composition in a subject subjected to an irradiation program, the composition comprising one or more thermal neutron capture agents, the neutrons having been generated by non-elastic collisions between a primary beam of charged particles and nuclei in the subject, wherein the charged particles consist of any one or more of protons, deuterons, tritons and heavy ions, the irradiation program comprises at least one period of irradiation with a beam duration that includes beam-on periods and beam-off periods;
an energy gate configured to apply at least one predefined energy window or filter such that the system accepts only detection events in the one or more detectors resulting from gamma-rays with an energy indicative of selected (e.g. principal or sole) gamma-rays arising from the capture of thermal neutrons by the one or more thermal neutron capture agents, wherein the thermal neutrons are neutrons with energies below approximately 0.4 eV;
a timing gate configured to receive beam data indicative of start or end of the respective beam-on periods and to generate from the beam data, and apply, a timing window configured such that the system rejects or ignores detection events in the one or more detectors resulting from at least prompt gamma-rays produced in non-neutron capture events; and
an output for outputting data indicative of the accepted detection events (such as to a data analysis system configured to determine thermal neutron radiation dose or a dose map, whether comprised within the system or external thereto).

The system may include a data analysis system, configured to determining a thermal neutron radiation dose received by the subject during the irradiation program from at least the accepted detection events or to determine a dose map of the thermal neutron radiation received by the subject from at least the accepted detection events.

The data analysis system may additionally be configured to receive beam data indicative of primary beam position (i.e. within the subject) and primary beam energy corresponding to the respective accepted detection events, and to determine the thermal neutron radiation dose or the dose map additionally based on the beam data.

It should be noted that applying the at least one predefined energy window can be performed online (i.e., as the detection events are being collected) or offline (i.e., after data collection, by filtering the detection events). Likewise, applying the timing window can be performed online or offline. Thus, in various embodiments, one or both of applying the energy window and applying the timing window are performed online, and in other embodiments one or both of applying the energy window and applying the timing window are performed offline.

The one or more detectors may be sensitive to the angle of arrival of gamma-rays, which is especially advantageous in determining a dose map.

The system may constitute or include a data logger or data analysis device configured to determine the radiation dose of the radiation received by the subject during the irradiation program from the counted gamma-rays or a dose map of the radiation received by the subject from the counted gamma-rays.

The energy gate may comprise first and second comparators (e.g. linear op amps or comparators with Schmitt trigger inputs).

The system may include a pileup rejector configured to rejecting pileup in output signals from the one or more detectors.

The timing gate may be configured to apply a timing window for rejecting the prompt gamma-rays by rejecting gamma-rays that arrive in the respective sensitive volumes from the start of each beam-on period to ending approximately 11 ns, approximately 12 ns, or from 10 to 12 ns, after the end of the respective beam-on period.

The timing gate may be configured to apply a timing window that also rejects detection events in the respective sensitive volumes attributable, based on timing, to fast neutrons.

The timing gate may be configured to apply a timing window that also rejects detection events in the respective sensitive volumes attributable, based on timing, to neutrons with energies between 0.4 eV and 1 MeV.

The timing gate may be configured to apply a timing window that rejects detection events in a detection period that has a ratio of true positive detection events to false positive detection events, ascertained empirically or by simulation, to be less than 1.4 or less than 1.5, wherein a true positive detection event is an event that satisfies the timing window and the energy window and arises from a neutron capture event, and a false positive detection event is an event that satisfies the timing window and the energy window and arises from other than a neutron capture event.

The system may include a shield arranged to at least partially shield some or all of the one or more detectors (and in particular the scintillators thereof) and comprising single or multiple layers of neutron absorbing and/or scattering material, wherein the material is different from the one or more neutron capture agents. As discussed above, the shield may optionally also be disposed over the side or sides of the one or more detectors.

If the neutron capture agent is or includes a ¹⁰B-based agent, the shield is most desirably arranged to shield both the fronts and sides of the one or more detectors. The shield typically comprises an individual shield for each detector. The neutron absorbing and scattering material are selected based on their neutron absorption and scattering cross-section and are thick enough to block or scatter most of the neutrons, but thin enough not to absorb too high a fraction of the gamma-rays due to neutron capture. The neutron absorbing or scattering material may comprise polyethylene, paraffin, cadmium, gadolinium, boron and/or hafnium.

In some examples, the one or more neutron capture agents are ¹⁰B-based and/or ¹⁵⁷Gd-based. For example, the one or more neutron capture agents may be ¹⁰B-based such that the gamma-rays have an energy of 478 keV, and/or ¹⁵⁷Gd-based such that the gamma-rays have an energy of 79.5 keV, 182 keV, 6.75 MeV, 7.86 MeV and/or 7.94 MeV.

In some examples, the neutron capture agent is ¹⁰B-based and the one or more detectors comprise any one or more of CdTe, CZT and LaBr₃:Ce detectors. The beam duration may be of from 1 to 10 *µ*s, or of approximately 1 *µ*s. The system may include a thermal neutron shield arranged to shield some or all of the one or more detectors and of a thermal neutron absorbing material comprising natural Cd, natural Gd and/or natural Hf.

In other examples, the neutron capture agent is ¹⁵⁷Gd-based and the one or more detectors comprise any one or more of LSO:Ce, BGO and PbWO₄ detectors. The beam duration may be of from 10 *µ*s to 100 ms. The primary beam may be a carbon ion beam and the beam duration approximately 1 ms, or the primary beam may be a helium ion beam and the beam duration approximately 10 *µ*s. The system may include a thermal neutron shield arranged to shield some or all of the one or more detectors and of a thermal neutron absorbing material comprising natural B and/or natural Hf.

It should be noted that any of the various features of each of the above aspects of the invention can be combined as suitable and desired.

### Brief Description of the Drawing:

In order that the invention may be more clearly ascertained, embodiments will now be described by reference to the accompanying drawing, in which:
Figure 1 is a schematic view of a patient recumbent on a couch of a Neutron Capture Enhanced Particle Therapy (NCEPT) irradiation system having a tumour irradiated by a beam of particles generated by the irradiation system.
Figure 2 is a schematic view of a dose quantification system according to an embodiment of the present invention.
Figure 3 is a schematic representation of beam intensity *I*_{B} (%) against time t, depicting the micro-structure of the beam of the NCEPT irradiation system of Figure 1.
Figure 4 is a schematic plan view of a simulated target phantom/detector geometry, used to model various dose quantification systems according to embodiments of the present invention.
Figure 5 is a plot of beam intensity I_{B} (%) against time t (ns), and depicts the micro-structure of primary beam modelled to test the dose quantification systems of Figure 4.
Figure 6A shows, in its upper register, depth-dose profiles for simulated ¹²C ion beam used to test the dose quantification systems of Figure 4, and, in its lower register, the energy spectrum used to generate the ¹²C ion beam.
Figure 6B shows, in its upper register, depth-dose profiles for simulated ⁴He ion beam used to test the dose quantification systems of Figure 4, and, in its lower register, the energy spectrum used to generate the ⁴He ion beam.
Figures 7A and 7B present two-dimensional spectrograms of neutron arrival time *t*ₙ (ns) in a neutron capture insert (NCI) region, following irradiation by polyenergetic ¹²C and ⁴He ion beams, respectively, plotted as neutron energy *E*ₙ (eV) versus neutron arrival time *t*ₙ (ns).
Figures 8A to 8F present spectrograms of photon detector volume arrival time *t*_{γ} (ns) as a function of gamma-ray energy *E*_{γ} (MeV) for, respectively, a ¹²C ion beam with no NCI, a ⁴He ion beam with no NCI, a ¹²C ion beam with a ¹⁰B NCI, a ⁴He ion beam with a ¹⁰B NCI, a ¹²C ion beam with a ¹⁵⁷Gd NCI, and a ⁴He ion beam with a ¹⁵⁷Gd NCI.
Figures 9A to 9F present spectrograms of neutron detector volume arrival time *t*ₙ (ns) as a function of the neutron energy *E*ₙ (MeV) for, respectively, a ¹²C ion beam with no NCI, a ⁴He ion beam with no NCI, a ¹²C ion beam with a ¹⁰B NCI, a ⁴He ion beam with a ¹⁰B NCI, a ¹²C ion beam with a ¹⁵⁷Gd NCI, and a ⁴He ion beam with a ¹⁵⁷Gd NCI.
Figures 10A and 10B present plots comparing the different detector material sensitivity (upper registers) and *R_{TF}* (lower registers), for ¹⁰B and ¹⁵⁷Gd NCIs, respectively, as functions of temporal mask Π(*t*) (ns), for realistic detector models following irradiation by a ¹²C ion beam.
Figures 11A and 11B present plots of sensitivity (upper registers) and the ratio of true / false positives (lower registers) as a function of temporal mask Π(*t*) (ns) for the two best detector materials for ¹⁰B and ¹⁵⁷Gd NCIs, respectively, following irradiation by a ¹²C ion beam for a range of total irradiation (i.e. beam duration) periods, for photons only.
Figures 12A and 12B present plots of sensitivity (upper registers) and the ratio of true / false positives (lower registers) as a function of temporal mask Π(*t*) (ns) for the two best detector materials for ¹⁰B and ¹⁵⁷Gd NCIs, respectively, following target irradiation by a ¹²C ion beam for a range of total irradiation (i.e. beam duration) periods and for all detected events.
Figures 13A and 13B present plots of sensitivity (upper registers) and the ratio of true / false positives (lower registers) as a function of temporal mask Π(*t*) (ns), for events recorded in realistic detectors using both no front shielding and various front shielding materials (viz. Cd, Gd, B and Hf) with high thermal neutron cross-section, for ¹⁰B and ¹⁵⁷Gd NCIs, respectively, following target irradiation by a ¹²C ion beam.
Figures 14A, 14B and 14C are plots of counts (N) versus ¹²C ion beam energy (keV), both with and without a boron NCI (Figure 14A), without a boron NCI (Figure 14B), and with a boron NCI (Figure 14C).
Figures 15A, 15B and 15C are plots of counts (N) versus ⁴He ion beam energy (keV), both with and without a boron NCI (Figure 15A), without a boron NCI (Figure 15B), and with a boron NCI (Figure 15C).

### Detailed Description of Embodiments

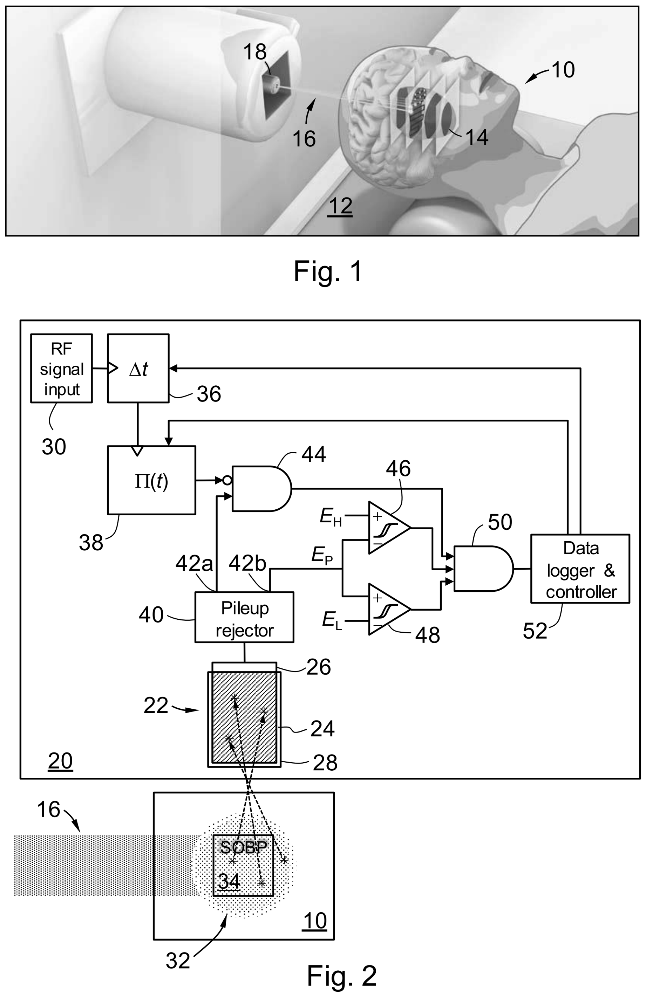

Figure 1 is a schematic view of a subject 10 recumbent on a couch 12 and having a tumour 14. The subject 10 is irradiated by a beam 16 of primary particles (e.g. protons, helium, carbon ions, etc.) generated by an Enhanced Particle Therapy (NCEPT) irradiation system. Beam 16 is emitted by treatment nozzle 18, having been generated by, in this example, a synchrotron accelerator (not shown).

As explained in WO 2019/051557, according to NCEPT, the subject 10 is administered with a dose of a thermal neutron absorbing nuclide such as a composition containing ¹⁵⁷Gd and/or ¹⁰B; the composition is selected to be preferentially absorbed by the tumour 14.

The depth of penetration of the primary particles in the subject is controlled by controlling the beam energy and shape, and thereby to locate the Bragg peak of the beam as desired relative to (and within) the desired target volume.

The subject is then irradiated with the beam 16 of primary particles in a desired scan pattern, depth, duration, beam energy, etc. according to a previously established irradiation program comprising one or more periods of irradiation (or 'beam on'), generally of uniform duration, each followed by a period without irradiation (or 'beam off'). This may include moving couch 12-and hence the target volume-between or during the period of irradiation, while minimizing subject movement.

During irradiation, a fraction of the primary particles in beam 16 undergo non-elastic collisions with nuclei in the subject 10, resulting in the production of a range of nuclear fragments, including short-range, high-LET charged particles and neutrons which are emitted from the point of collision within the subject, and which deposit their energy in the region surrounding the path of beam 16. Some of the neutrons so produced are absorbed by the thermal neutron absorbing nuclide of the administered composition, resulting in the production of energetic charged particles with high relative biological effectiveness that irradiate the target volume and hence tumour 14.

Figure 2 is a schematic view of a dose quantification system 20 according to an embodiment of the present invention, shown with subject 10 being irradiated by the beam 16 of primary particles extracted from a synchrotron as described above. Dose quantification system 20 is configured to be employed with or as a part of the NCEPT irradiation system, and is configured to determine both the direct ion dose and the dose due to neutron capture, including discriminating between photons resulting from neutron capture and those originating from other processes.

Dose quantification system 20 has a gamma-ray detector 22 for detecting gamma-rays emitted by the subject 10 (and which-in this embodiment-includes a scintillator 24, and a photodetector 26), and an RF signal input 30 for receiving an RF pulse train from the synchrotron. This RF pulse train comes directly from the synchrotron and is indicative of the beam micro-structure. There is a transmission delay, but the pulse train is rigidly periodic so subsequent triggering in system 20 can be adjusted accordingly to allow for that delay.

Alternatively, the pulse structure can be extracted directly from the primary beam (as opposed to the RF signal from the synchrotron, cyclotron, etc). This can be achieved by inserting a hodoscope into the beam's path as the beam exits nozzle 18. A hodoscope can comprise, for example, a fibre optic detector (comprising a plastic scintillator) coupled to a single silicon photomultiplier; the output of such an arrangement (which has a low detection efficiency but fast rise-time) can be used to provide a direct timing signal to the data acquisition system, independent of the synchrotron or cyclotron's RF signal.

Gamma-ray detector 22 is located so as to detect gamma-rays emitted by the subject in response (directly or indirectly) to the irradiation, and in particular to detect gamma-rays emitted in the cloud 32 of thermal neutrons that result from collisions between the beam 16 of primary particles and nuclei in the subject 10-some of which collisions will occur in and adjacent to the target volume. The beam 16 is generated so as to have a spread-out Bragg peak (SOBP), which is done by varying the energy of the beam 16, thereby varying-or spreading out-the depth of the Bragg peak. Region 34 within the thermal neutron cloud 32 indicates the spread-out Bragg peak (SOBP) of beam 16. SOBP 34, which will typically coincide (at least approximately) with or determine the target volume, may be either passively shaped (viz. by placing a ridge filter in the path of the beam 16), or delivered dynamically by 'painting' the target volume with a monoenergetic primary particle beam, slice by slice.

Figure 2 depicts one gamma-ray detector 22, but it should be understood that, in a practical system, system 20 will generally include a plurality of gamma-ray detectors, arranged-for example-in a ring or cylindrical formation around the subject 10, or in a partial ring or partial cylindrical formation (such as mounted on a C-shaped gantry around the subject 10).

The scintillator 24 of gamma-ray detector 22 is selected to have a high detection efficiency for the gamma-ray energies of interest. This is achieved through choosing a high density/high atomic number scintillator material, with a low self-absorption at the energies of interest. In this embodiment, scintillator 24 is an LSO (Lu₂SiO₅:Ce) scintillator, but in an alternative embodiment detector 22 may be a LaBr₃:Ce based detector.

Though not essential, it is advantageous that the detector 22 be sensitive to the angle of arrival of the detected gamma-rays. For example, in embodiments of system 20 with one or more detectors 22, a pre-therapy image may be constructed using system 20 by detecting radiation (including the angle of arrival) emitted by a radiotracer analog of the neutron absorbing agent administered to the subject, where the analog has comparable pharmacokinetics to those of the intended neutron absorbing agent(s). For example, when boron is used in the form of ¹⁰B-BPA as the neutron capture agent, suitable radiotracers include ¹⁸F-BPA, ¹³¹I-BPA, ¹⁸F-FET (all BPA boronophenylalanine analogs); when gadolinium is used in the form of Gd-TPP-DOTA as a neutron capture agent, suitable radiotracers include Gd-TPP-DOTA (an MRI contrast agent), or that radiotracer with the Gd replaced with ⁴⁴Sc, ¹⁷⁷Lu, or ⁶⁸Ga.

In this procedure, conducted for treatment planning, the radiotracer analog is injected into the subject, and system 20 is used to determine (i) the spatial distribution of the radiotracer (and hence the expected spatial distribution of the neutron capture agent(s)), and (ii) the contrast ratio that can be achieved between tumour, lesions or other pathological tissue, and non-cancerous or normal tissue. This information can be input into a treatment planning system, so that the extra dose due to neutron capture can be estimated, and the distribution of neutron captures in the subject during irradiation can be predicted. Finally, system 20 can then be used to quantify the neutron capture distribution (e.g. at specific positions and directions with a single collimated detector, or in the entire volume with a multi-detector collimated imaging system) to verify it against the prediction of the treatment planning system.

Similarly, embodiments of system 20 with a plurality of detectors 22 can be configured to use the detected gamma-rays from neutron capture to estimate the distribution of the neutron capture events and provide an accurate dose map. Such embodiments do so using the angle of arrival of the detected gamma-rays and back-projecting the response of the detectors.

Advantageously, scintillator 24 has a low cross-section for interaction with neutrons or, more precisely, to being activated when exposed to neutrons (as such interactions will increase the background). In this embodiment, however, this potential problem is optionally addressed by including in system 20 a multi-layered neutron shield 28 disposed over the front face and, in this embodiment, over the sides of detector 22. (Note that shield 28 is not shown to scale in the figure.) Neutrons can be almost entirely absorbed or scattered by layers of shielding with high thermal neutron cross section and high fast neutron scattering cross-section, without significantly attenuating neutron capture gamma photon fluence-provided that the shielding material is distinct from the neutron capture isotope used during NCEPT treatment. Shield 28 is configured according to these criteria and, in this embodiment, comprises polyethylene, paraffin, cadmium, gadolinium, boron, boron-carbide and/or hafnium (subject to the choice of neutron capture isotope used during the NCEPT treatment), with a thickness selected so as to be (i) thick enough to block or scatter away most neutrons, but (ii) thin enough not to absorb too high a fraction of the neutron capture gamma-rays (being the gamma-rays of interest). Shield 28 of this embodiment has up to six layers, each layer with a thickness of approximately 1 mm to 2 cm, but it would be a straightforward matter to modify or optimize these thicknesses according to application and the aforementioned selection criteria. Furthermore, natural forms of the shielding materials are contemplated (i.e. without enrichment of any of their constituent isotopes). Enriched forms of those materials (i.e. enriched in the most absorbent or scattering isotope(s)) may be used, but the expense of enrichment may not be justified by the potential benefit.

It should be noted that in some applications system 20 need not include shield 28. For example, the problem of neutron activation of detector 22 (which can increase background radiation) typically becomes greater as the detector ages. Also, neutrons start to arrive at the detector 22 in significant numbers only after 10⁴ ns. If the former is not a concern or events detected more than 10⁴ ns after the start of a beam on period are excluded, shield 28 may be deemed unnecessary.

It is envisaged that shield 28 will satisfactorily serve to reject neutrons from the detector 22, but alternatively (or additionally) this may be effected by scattering at least some of the problematic neutrons before they can reach detector 22. This can be done by locating a suitable material between the subject and the detector 22, selected to transmit the gamma-rays of interest with sufficient efficiency so as not to compromise the measurement, but to scatter such neutrons before they reach the detector. The appropriate material and its dimensions can be determined by straightforward experimentation or simulation. Many polymers (and in particular low density polymers) meet these criteria.

Photomultiplier 26 of detector 22 is selected to handle high count rates augmented, as described below, by the use of pile-up rejection circuitry. In this embodiment, photomultiplier 26 is a silicon photomultiplier.

The RF pulse train indicates when the beam 16 from the synchrotron is on and off, that is, is irradiating or is not irradiating the subject 10, so that data collection can be controlled relative to the periods of irradiation of the irradiation program. In particular, as described in greater detailed below, system 20 controls data collection to occur when the subject 10 is not being irradiated, that is, generally between successive periods of irradiation, and to commence after a preselected period from the completion of each period of irradiation (measured, for example, from the start or end of the respective period of irradiation).

System 20 also includes a detection window start delay 36 and a timing gate 38. Timing gate 38 is configured to control when detection events from detector will be rejected on the basis of the timing of the detection event in detector 22 relative to beam on and beam off periods (as discussed in detail below). Detection window start delay 36 ensures that this is coordinated with the beam on and beam off periods: detection window start delay 36 receives as input the aforementioned RF pulse train and a trigger signal fed through the detection system, and adds a suitable signal propagation delay to timing gate 38 so that timing gate 38 can generate a temporal mask Π(*t*) for each beam on period (being the period in which detection events are rejected) with a beginning that coincides with the start of the respective beam on period. This temporal mask Π(*t*) is of a preselected duration. Timing gate 38 outputs a low signal when the temporal mask Π(*t*) is to be enforced (i.e. detection events are to be rejected), and a high signal otherwise.

It should be noted that system 20 can-in some embodiments-be configured as a data logger, that is, as a high bandwidth pulse detection system that records all gamma-ray detection events in list mode, where each recorded detection event includes the arrival time (i.e., time stamp), the energy of the event, and the identity of the detector that recorded the detection event. In such embodiments, detection events can then be processed offline after the delivery of the treatment.

Data collection continues, relative to each beam on period, for a preselected data collection window *DCW.* Data collection window *DCW* is typically set to extend for as long after each beam-on period ends as it is expected (or it has been found) that the gamma-rays of interest will be received or can be detected efficiently, which depends on when and for how long thermal neutrons (as defined herein) interact with the neutron capture agent(s). In this embodiment, data collection window *DCW* has an end point at least approximately 10^{5.8} ns, at least approximately 10⁶ ns, or between 10^{5.8} ns to 2 × 10⁶ ns, after the end of the respective beam-on periods; this is substantially greater than the beam off periods, with the consequence that data is collected continuously until a period equal to the collection window *DCW* after the end of the final temporal mask Π(*t*).

It should be noted that data collection window *DCW* may be specified either as a data collection duration (running from the end of the beam on period), or as data collection start and data collection end. Data collection start may fall within a temporal mask, but data collection will not effectively commence until the termination of the temporal mask Π(*t*), as detection events will be rejected while the temporal mask Π(*t*) is enforced by timing gate 38.

System 20 includes a pulse pileup rejector 40 (such as in the form of a deconvolution filter), which receives the output pulses of gamma-ray detector 22 and has active circuitry configured to deconvolve the incoming pulse waveform by the combined impulse response of the scintillator and associated electronics to convert it into a sequence of short impulses with peak amplitudes proportional to the energy deposited in the detector. The pulses from the scintillator will exhibit a fast rise time and a (relatively) slow decay time (which will total, in the example of an LSO detector, approximately 35 ns). Hence, if multiple events occur within a short period they can overlap (and linearly add together). The pulse pileup rejector 40 deconvolves this using a model of the impulse response of the scintillator and preamplifier electronics, converting the pulses back into a train of Dirac delta impulses (i.e. of very short duration with pulse height proportional to deposited energy). The second output 42b of pulse pileup rejector 40 outputs signals indicative of the peak energy *E*_{P} of each pulse.

Pulse pileup rejector 40 is employed whether system 20 is configured for online or offline processing.

System 20 includes a first AND gate 44, which receives the mask signals from timing gate 38 and the pulses from gamma-ray detector 22 passed by pileup rejector 40, and outputs only those pulses received when the gate signal from timing gate 38 is low.

System 20 includes first and second comparator devices 46, 48 with Schmitt trigger inputs (which are faster than standard linear op-amps), configured to provide an energy gate. Together they define an energy window (of energy *E*_{L} to energy *E*_{H}) and thereby select the gamma-rays of interest: the prompt gamma-rays arising from neutron-capture by the thermal neutron absorbing nuclides. First comparator device 46 receives at its non-inverting input a signal indicative of a maximum desired energy *E*_{H} of the pulses outputted by gamma-ray detector 22, and a signal indicative of the peak energy *E*_{P} of each pulse at its inverting input. Second comparator device 48 receives the signal indicative of the peak energy *E*_{P} of each pulse at its non-inverting input and a signal indicative of a minimum desired energy *E*_{L} for the pulses outputted by gamma-ray detector 22 at its inverting input. Thus, first comparator device 46 and second comparator device 48 both have high outputs only when the energy *E*_{P} of a pulse from gamma-ray detector 22 is within the energy window defined by *E*_{H} and *E*_{L}*.*

System 20 includes a second AND gate 50, which has three inputs and which receives the outputs of first AND gate 44, first comparator device 46 and second comparator device 48. Consequently, second AND gate 50 has a high output only when a pulse is received from gamma-ray detector 22 that falls within both the data collection timing window and the peak energy window (i.e. between *E*_{H} and *E*_{L}).

System 20 includes a data logger and controller 52, configured to receive and log the signals received from second AND gate 50. Data logger and controller 52 records the number of detections within each beam micro-pulse (i.e. each individual data collection window) and the time of arrival of each beam micro-pulse, so that each micro-pulse can be correlated with the treatment plan. (This is more important for embodiments of system 20 that are used with a raster-scanned pencil primary beam, where the beam is stepped around in space and in energy; in a passively scattered particle therapy system the beam is the same for the duration of the treatment fraction.) Data logger and controller 52 also receives beam data (indicative of, for example, the x, y position of the beam 16 and the beam energy) corresponding to each beam on period, which are stored in a manner that allows the detection events recorded by data logger and controller 52 to be associated with the beam data (such as by employing timestamps) and hence with the instantaneous location of irradiation by the beam 16 within the subject.

Data logger and controller 52 is also configured to provide a trigger signal to detection window start delay 36, and is user-operable to select and set the values of the preselected temporal mask Π(*t*) and data collection window *DCW.* Data logger and controller 52 may be incorporated into a data acquisition system. The data logger and controller 52 (or such a data acquisition system) may be additionally configured to perform data analysis including determining radiation dose, and/or to transmit the logged data to a data analysis system or computing device (not shown)-whether provided as a part of system 20 or as an external system/device. In this embodiment, the data analysis is performed offline with a data analysis system.

That data analysis, whether performed by data logger and controller 52, the aforementioned data analysis system or another computing device, involves determining the radiation (i.e. thermal neutron) dose received by the subject and/or a dose map (whether presented as neutron capture distribution or dose distribution), based on the logged detection events. This is done using conventional techniques. For example, beam 16 may be a raster-scanned energy-modulated pencil beam (which, as mentioned above, essentially 'paints' the target volume with the primary ion dose one voxel at a time). The analysis thus involves correlating the x, y positions and energy of the beam with the detected neutron capture photons, so that the detection event can be correlated with instantaneous beam position. The data collected by system 20 are then used to determine neutron capture or dose distribution, and neutron radiation dose. Dose is determined as deposited energy per unit mass (Gy), which is then be converted to gray equivalent (GyE) or the biological dose, as desired.

Advantageously, this analysis may additionally employ pre-irradiation CT or MRI anatomical (structural) imaging for treatment planning, a pre-irradiation estimate of the distribution of the neutron capture agent(s) (measured using PET or SPECT imaging and a neutron capture agent radiotracer analog, discussed above), and post-irradiation PET imaging to indirectly confirm the spatial ion dose distribution. The expected neutron dose is calculated based on the treatment plan (which uses the CT or MRI images and the neutron capture agent distribution estimate to calculate the beam parameters that will achieve a desired dose distribution, including both the ion and neutron-capture dose). The total delivered dose is estimated based on the PET images captured after irradiation (which give an indirect estimate of the ion dose distribution) and the neutron capture distribution determined by system 20. If system 20 has a single detector 22 (or a limited number of detectors), it may not be possible to estimate a full (or high resolution) 3D neutron capture distribution, in which case the results from system 20 are validated against the expected neutron capture distribution from the treatment plan. This can be facilitated by comparing the results with previous plans/results, such by applying machine learning to neutron capture agent distribution estimation and structural information to obtain a good estimate of the true neutron capture distribution from limited projection angles.

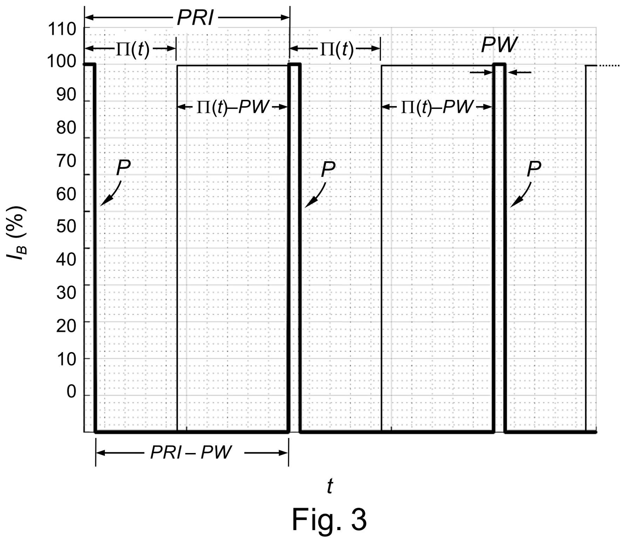

Figure 3 is a schematic representation of beam intensity *I*_{B} (%) against time t, depicting the micro-structure of the beam 16 [Refs. 29, 30]. As may be seen, the irradiation comprises a repeating pattern that continues for the entire irradiation period (or 'beam duration'). The pattern comprises a 'beam on' period with a pulse P of width *PW,* repeated with a pulse repetition interval *PRI* such that the successive 'beam off' periods have a duration of *PRI - PW.* Each data collection period is subject to the data collection window *DCW* and temporal mask Π(*t*), as described above. In this example, data collection window *DCW* commences at the termination of temporal mask Π(*t*). The beam duration thus refers to the period from the start of the first beam on period to the end of the last beam on period.

As discussed above, in NCEPT, the subject 10 is administered with a dose of a composition containing one or more thermal neutron absorbing/capture nuclides (e.g. ¹⁵⁷Gd and/or ¹⁰B). Furthermore, the irradiation program may differ according to location and nature of the target volume, as well as other clinical indications. In broad terms, system 20 is configured to apply a temporal mask of at least the beam-on period *(PW)* plus the period in which non-neutron-capture prompt gamma-rays are emitted, estimated to be 11 ns, depending on the characteristics of the detector. This minimal temporal mask is most suitable for a detector that approaches the performance of an ideal gamma-ray detector (i.e. having perfect absorption, infinite energy resolution, and no dead time), but in practice, a greater temporal mask will provide better results so be more desirable.

However, in practice 'fast neutrons' (E > 1 MeV) and 'intermediate energy neutrons' (for present purposes defined as 0.4 eV ≤ *E* ≤ 1 MeV) can potentially deposit enough energy in detector 22 to satisfy the energy window for neutron-capture prompt gamma detection (viz. energy *E*_{L} to energy *E*_{H}). Intermediate energy neutrons arrive at the detector in relatively low fluences and over a longer timescale compared to fast neutrons and thermal neutrons (for present purposes defined as E < 0.4 eV), but cannot be effectively blocked by shielding 28 without compromising detector sensitivity. Shielding against fast neutrons is partially achieved by using a scattering material, but the remaining fast neutrons arriving at the detector will do so within approximately 49 or 50 ns of the end of the beam pulse (providing that the detector itself does not become activated with long-lived radio-isotopes), so the detection of fast neutrons can be greatly reduced by using a temporal mask Π(*t*) of up to 50 ns, with the benefit increasing as the temporal mask increases.

Hence, system 20 is advantageously configured to apply a temporal mask of at least the beam-on period (PW) plus approximately the fast neutron arrival time. In a particular example, the preferred temporal mask is the beam-on period (PW) plus 49 ns.

The duration of the data collection temporal window *DCW* is selected according to the period in which the majority of gamma-rays due to thermal neutrons (as defined above) arrive in detector 22. It has been found that, as thermalisation takes time, this occurs in an approximately 12 ns to 10⁶ ns window from the commencement of a beam on period, with almost none appearing outside that window. Hence, in principle, system 20 can employ a data collection temporal window *DCW* of from 12 ns to 10⁶ or 2 × 10⁶ ns after commencement of the beam-on period. However, as shown below the majority of photons resulting from neutron capture arrive at detector 22 at least 60 ns later than photons created by other processes, so system 20 is configured to apply a temporal mask Π(*t*) that extends at least 60 ns beyond beam off (i.e. Δ*t* ≥ *PW* + 60 ns), and in the particular example shown in Figure 1, system 20 is configured to apply a temporal mask of Π(*t*) = *PW +* 60 ns.

In addition, it has been determined that the duration of the temporal mask Π(*t*) affects the number of false detection events (termed 'false positives' herein), which is in turn also affected by total irradiation time (i.e. beam duration), neutron absorption/capture agent, the type of gamma-ray detector 22 and the material(s) used for shield 28. System 20 is configured to operate in regimes that take these factors into account and in particular to have a high or optimized ratio of true detection events (termed 'true positives' herein) to false detection events. Examples of these regimes-with individual options in ranked order-are listed in Table 1A (for a carbon ion beam) and Table 1B (for a helium ion beam).

It is envisaged that, if the composition comprised a mixture of ¹⁰B and ¹⁵⁷G, a detector 22 would be selected that works well for both gamma-ray energies (e.g. LSO), though it is recognized that this selection would involve some compromise. The thermal neutron shielding material of optional shield 28 would also have fewer good options (such as to Cd or Hf). The optimal beam duration would also represent a compromise; for example, with a carbon ion beam a 10 *µ*s

**Table 1A: Agent, Detector, Shielding Material & Beam Duration, Carbon Ion Beam**

| **Neutron Capture Agent** | **Detector** | **Shielding Material** | **Beam Duration** |
|---|---|---|---|
| ¹⁵⁷Gd | LSO:Ce | natural B | 1 ms |
| | BGO | natural Hf | 1 *µ*s |
| | PbWO₄ | none | 10 ms |
| | | | 100 ms |
| ¹⁰B | CdTe | none | 1 *µ*s |
| | CZT | natural Cd | 10 *µ*s |
| | LaBr₃:Ce | natural Gd | |
| | | natural Hf | |

**Table 1B: Agent, Detector, Shielding Material & Beam Duration, Helium Ion Beam**

| **Neutron Capture Agent** | **Detector** | **Shielding Material** | **Beam Duration** |
|---|---|---|---|
| ¹⁵⁷Gd | LSO:Ce | natural B | 10 *µ*s |
| | BGO | natural Hf | 1 ms |
| | PbWO₄ | none | 10 ms |
| | | | 100 ms |
| ¹⁰B | CdTe | none | 1 *µ*s |
| | CZT | natural Cd | 10 *µ*s |
| | LaBr₃:Ce | natural Gd | |
| | | natural Hf | |

beam duration would be acceptable for both agents while, with a helium ion beam, a 10 *µ*s beam duration would be acceptable for ¹⁰B and optimal for ¹⁵⁷G. A timing or temporal mask Π(*t*) of 60 ns (i.e. extending 49 ns after the end of each beam on period) would be acceptable for both.

Alternatively, system 20 could include a plurality of detectors 22 (optionally with thermal neutron shields 28), with some optimized for ¹⁰B and some optimized for ¹⁵⁷G.

### SIMULATED EXAMPLES

Preferred characteristics of system 20 were determined and evaluated using a series of simulations, in a two part study.

Firstly, a Monte Carlo simulation model was constructed comprising a target phantom irradiated by a helium or carbon ion beam with a spread out Bragg peak (SOBP) with a 60 mm depth range. Neutrons are predominately created with initial momentum parallel to the beam [Refs. 14, 17], so detectors were simulated as positioned proximal to the Bragg peak and orientated towards the region of interest, to minimize the number of neutrons interacting directly with those simulated detectors.

Three target phantoms were used in the simulations:
i) a solid homogeneous block of poly(methyl methacrylate) (PMMA);
ii) a solid block of PMMA with a pure ¹⁰B insert located at the distal end of the SOBP;
iii) a solid block of PMMA with a pure ¹⁵⁷Gd insert located at the distal end of the SOBP.

The energy and time of arrival (relative to the time of primary particle generation) of all neutrons entering the insert region of each phantom were scored; the time of arrival (also relative to the time of primary particle generation) and energy of photons that exit the phantom via a band parallel to the insert region were also scored. Based on these results, a temporal mask and energy window suitable for neutron capture discrimination were determined.

The temporal mask and energy window were applied to all particles arriving at an ideal detector (i.e. simulated as having perfect absorption, infinite energy resolution, and no dead time) and the numbers of true and false positive and negative classifications (where the ground truth is the known origin of each detected event) were counted. True positive and true negative detections were defined as those where the detection is correctly classified as neutron capture or non-capture, respectively.

A false positive was defined to be a detection that satisfied the timing and energy acceptance windows but was not related to a neutron capture event. A false negative was defined to be a detection that was related to neutron capture but failed to satisfy the timing and/or energy acceptance windows. The former could be due to late photon arrival, owing to unusually rapid or slow neutron thermalisation prior to capture, leading to long but low-probability tails in the temporal distribution of neutron captures. Failure to satisfy the energy acceptance window could be due to Compton scattering of the emitted photon in the phantom (resulting in a lower energy and an altered photon trajectory), Compton scattering in the detector (in the case of the realistic detector models) followed by the escape of the scattered photon (resulting in only part of the photon's energy being deposited in the detector), or the detection of particles other than photons (e.g. fast neutrons, protons or other fragments) in the detector.

For the final part of the study, realistic detector simulation models were used, and the same evaluation of true/false positive/negative detection was performed. Several alternative detector materials were evaluated, including direct-detection CdTe, CZT (for boron neutron capture) and LaBr₃:Ce, LSO (Lu₂SiO₃:Ce), BGO and PbWO₄ scintillator-based detectors (for both boron and gadolinium neutron capture). These detectors were simulated both unshielded and with thin layers of several alternative thermal neutron shielding materials.

### Materials and Methods

Geant4 version 10.2.p03 was used for all simulations, as it has been previously identified as providing the best agreement with experimental fragmentation measurements in particle therapy [Refs. 18, 19, 20]. Electromagnetic interactions were modelled using the standard Geant4 physics option 3 model (G4EmStandardPhysics_option3), while the other physics models (including hadronic interactions) used in the simulation are listed in Table 2.

**Table 2: Physics models used in all simulations**

| **Interaction** | **Energy Range** | **Geant4 Model** |
|---|---|---|
| Radioactive Decay | N/A | G4RadioactiveDecayPhysics |
| Particle Decay | N/A | G4Decay |
| Hadron Elastic | 0-100 TeV | G4HadronElasticPhysicsHP |
| Ion Inelastic | 0-110 MeV | Binary Light Ion Cascade |
| | 100 MeV-10 GeV | BIC |
| | 9.99 GeV-1 TeV | FTFP |
| Neutron Capture | 0-20 MeV | NeutronHPCapture |
| | 19.9 MeV-100 TeV | nRadCapture |
| Neutron Inelastic | 0-20 MeV | NeutronHPInelastic |
| Neutron Elastic | 0 eV-20 MeV | NeutronHPElastic |
| | 20 MeV-100 TeV | hElasticCHIPS |
| Proton Inelastic | 0-9.9 GeV | Binary Cascade |

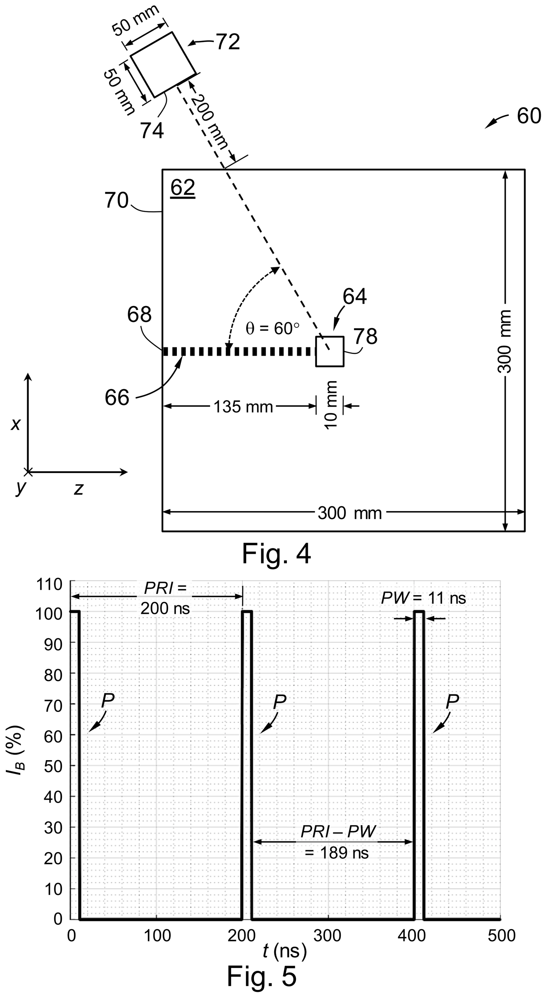

Figure 4 is a schematic, plan view of the target phantom geometry 60 (not to scale), as simulated. Each of the three simulated target phantoms 62 comprised a 300 × 300 × 300 mm³ PMMA cube, the first a homogeneous block of PMMA, while the second and third included a 10 mm cubic neutron capture insert (NCI) of pure ¹⁰B and pure ¹⁵⁷Gd, respectively, embedded with its centre at a depth of 140 mm within the PMMA phantom (hence at the distal end of the spread-out Bragg peak, described below), and centred laterally and vertically. The physical properties of the materials used in the simulations, including elemental compositions and density, were based on the library of standard materials defined by the National Institute of Standards and Technology (NIST) [Ref. 21].

Polyenergetic beams 66 of ¹²C and ⁴He were synthesised with energy ranges of 225-294 MeV/u and 113-156 MeV/u, respectively, with a spread-out Bragg peak extending 60 mm so as to create an approximately flat biological dose between depths of 85 mm and 145 mm in the phantom. (The procedure that was used to create a flat biological dose is described in Ref. [1].) The ion beams 66 were rotationally symmetric, with a 20 mm FWHM Gaussian beam profile, and were generated at the centre 68 of the front face 70 of the phantom 62, moving parallel to the z-axis of the phantom, with 10⁸ particles simulated for each beam and phantom combination. The 'point of beam entry' of the beam 66 (viz. the point of beam generation) was defined to be the origin (0, 0, 0) of the simulation coordinate system. The energy deposited in the target phantom 62 by each ion beam 66 was scored as a function of depth across the full width at tenth maximum of the ion beam 66 and normalised to the energy deposited at the point of beam entry 68.

### Neutron and gamma characterisation

For phantoms (ii) and (iii), that is, with an NCI 64, the energy and arrival time of neutrons entering the insert region 64 were recorded. A two-dimensional spectrogram illustrating neutron arrival time (horizontal axis) vs. kinetic energy (vertical axis) was constructed, with 50 logarithmically spaced temporal bins (from 0.1 ns to 10⁸ ns), and 50 logarithmically spaced energy bins (from 10⁻⁵ eV to 10¹⁰ eV). For each particle, time was measured relative to the instant at which the incident carbon or helium particle was generated at the surface 70 of the phantom 62.

The energy and time of arrival of photons and neutrons entering the detector were scored and an energy (horizontal axis) vs. photon arrival time (vertical axis) spectrogram was produced. Arriving photons were distributed into 1000 linearly spaced energy bins (each 10 keV in width, from 0 to 10 MeV), and 100 logarithmically spaced arrival time bins (from 0.1 ns to 10¹⁰ ns). Arriving neutrons were distributed into 130 logarithmically spaced energy bins (from 10⁻¹⁰ MeV to 10³ MeV), and 100 logarithmically spaced arrival time bins (from 0.1 ns to 10¹⁰ ns). Time was again measured relative to the instant at which the primary particle was generated.

### Detector material, beam on duration and shielding optimisation

The second part of the study determined the accuracy with which photons resulting from thermal neutron capture in the neutron capture insert could be distinguished from photons originating from other processes, and used several alternative detector models.

Referring to Figure 4, an ideal detector 72 was modelled as a simple geometric volume configured to record the identity, creating process, time of arrival and energy of any particle entering the detector volume. The detector volume was cubic, with dimensions of 50 mm × 50 mm × 50 mm. The detector 72 was positioned as shown in Figure 4, with the normal vector of its front face 74 oriented towards the centre 78 of the distal edge of the SOBP region in the phantom 62 (hence oriented towards the point with coordinates (0, 0, 145 mm). The front face 74 of the detector 72 was at a distance of 47.32 cm from the centre 78 of the distal edge of the SOBP, at an angle of *θ* = 60 deg (about the vertical- or *y*-axis) relative to the ion beam 66.

The realistic detector models used the same geometry as the ideal detectors. Table 3 lists the different detector materials used in modelling realistic detectors. LaBr₃, CZT, LYSO:Ce and CdTe are suitable for detecting the gamma photons from ¹⁰B neutron capture, as they provide the highest energy resolution, while the higher densities of LSO:Ce, BGO and PbWO₄ are more appropriate for detecting the higher energy photons emitted during ¹⁵⁷Gd neutron capture. (It was assumed that several scintillator crystals or semiconductor detectors were stacked and optically/electronically coupled to form a single detector.)

**Table 3: Detector materials, including a range of scintillators and two direct-detection materials.**

| **Material** | **Density (g/cm³)** | **Yield (ph/MeV)** | **Decay time (ns)** | **Energy resolution (%)** | **Emission *λ* (nm)** | **Data source** |
|---|---|---|---|---|---|---|
| LSO:Ce | 7.10-7.40 | 30000 | 40 | 10.5 | 420 | [22] |
| BGO | 7.13 | 8000-10000 | 300 | 9.7 | 480 | [23] |
| LaBr₃:Ce | 5.06 | 63000 | 16-25 | 2.2-2.6 | 380-385 | [24, 25] |
| PbWO₄ | 8.28 | 400-500 | 6/30 | 36 | 440/530 | [26, 27] |
| CdTe | 5.85 | N/A (direct) | N/A (direct) | 2.4 | N/A (direct) | [28] |
| CZT | 5.8 | N/A (direct) | N/A (direct) | 2-3 | N/A (direct) | [29] |

Figure 5 is a plot of beam intensity *I*_{B} (%) against time t (ns), and depicts the micro-structure of the beam during the first 500 ns, in a pattern that was repeated for the entire irradiation period. Total irradiation periods of 1 *µ*s, 10 *µ*s, 1 ms, 10 ms and 100 ms were simulated.

The beam's timing micro-structure was modelled as a sequence of pulses P with a period *PW* of 200 ns and a 'beam on' period *PW* of 11 ns (a duty cycle of 5.5%). A total of 10⁹ primary particles were used for each simulation, with the particles injected periodically at a constant rate during each nano-spill. For any particle depositing energy in the detector, the particle type, creating process, time of arrival, total deposited energy, and location(s) of energy deposition in the detector were scored; all individual energy depositions resulting from multiple-interaction events (e.g. multiple Compton interactions) were summed. The optical scintillation process was not modelled: doing so in Geant4 is possible but increases the execution time by several orders of magnitude, and is not important for present purposes. It was assumed that the area under the output pulse from the optical photodetector was proportional to the deposited energy.

The ion beams, target geometry and composition were the same as in the first part of this study, so a phase space record of the prompt gamma photons generated in the previous simulation (including the energy, time, position and responsible process of particle creation within the phantom) was used to drive this simulation, substantially reducing the required simulation time.

Based on the results presented in the first part of the study, an energy window and temporal mask were defined to differentiate between neutron capture photons and photons unrelated to neutron capture in the NCI region. Energy deposited into the detector during the masked-out intervals was not scored. Eight different temporal masks were investigated:
- 0 ns: no temporal mask applied;
- 11 ns: temporal masking during beam on period only;
- 11 ns + Tₚᵣₒₘₚₜ = 22 ns total: temporal masking during beam on period plus prompt gamma emission period (determined based on the outcome of the first part of the study); and
- 11 ns + Tₚᵣₒₘₚₜ + Tₙₑᵤₜᵣₒₙ = 30, 40, 50, 60, 70, 80 ns total: temporal masking during beam on period plus prompt gamma plus neutron emission period (determined based on the outcome of the first part of the study).

As discussed above, each temporal mask was applied from the start of each nano-spill and included the entirety of the irradiation ('beam on') interval. The following performance metrics were calculated for each irradiation interval, each ion beam (carbon and helium), and each detector model (ideal plus each of the realistic models):
- The sensitivity, defined as the number of true positive, false positive, true negative and false negative detections normalised by the number of photons reaching the detector; and
- The fraction of true positive, false positive, true negative and false negative detections, as defined above.

Using (1) raw true and false positive sensitivity performance metrics, and (2) the *true positives : false positives* ratio (*R_{TF}*), which is a metric for selectivity, a series of separate comparisons were performed to optimise different aspects of the detector design, as follows.

### Comparison of detector materials

The performance metrics were evaluated for each of the different detector materials across a range of temporal mask intervals with a fixed irradiation period. For the ¹⁰B NCI target, an irradiation period of 1 *µ*s was chosen based on the results of the first part of the study, since after this period the relative numbers of 511 keV and 2.23 MeV photons begin to increase, which make it difficult to discriminate between the background and the 478 keV photons from boron neutron capture. For the ¹⁵⁷Gd NCI target, the irradiation period is less critical to absolute sensitivity and selectivity; an irradiation period of 1 ms was selected. Irradiation periods were subsequently optimised, as follows.

### Comparison of irradiation durations

The effect of irradiation duration on sensitivity and *R_{TF}* for a range of temporal mask intervals were evaluated for the best-performing materials identified above: firstly, for only the photons depositing energy in the detector, and secondly, for all particles depositing energy in the detector. The chosen detectors for this comparison were the CdTe detector for the ¹⁰B NCI and the LSO detector for the ¹⁵⁷Gd NCI.

### Comparison of shielding materials

Utilising the best-performing detector materials (see above), viz. CdTe for the ¹⁰B NCI and LSO for the ¹⁵⁷Gd NCI, a range of different high thermal neutron cross-section front-face shielding materials were evaluated. The shield was a 50 mm × 50 mm × 1 mm layer of the evaluated material, applied to the front face only of the detector. For phantoms with a ¹⁰B NCI, (natural) gadolinium, cadmium and hafnium were evaluated as potential thermal neutron shielding materials, while for those with the ¹⁵⁷Gd NCI, (natural) boron, cadmium and hafnium were evaluated.

### Results

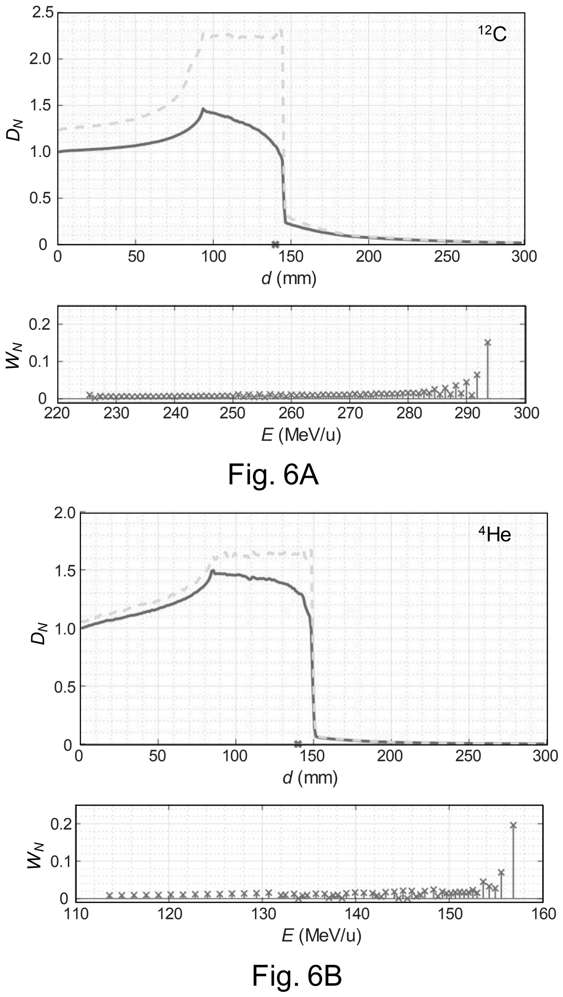

Figure 6A shows in its upper register depth-dose profiles (normalised to entrance dose) for the ¹²C ion beam, plotted as normalized dose *D_{N}* against depth in phantom d (mm); the solid curve indicates dose, while the dashed curve indicated predicted biological dose. The "x" at (140, 0) indicates the proximal face of the NCI region.

Figure 6A shows in its lower register the energy spectrum used to generate the ¹²C ion beam, plotted as normalized energy weight *W_{N}* against beam energy *E* (MeV/u).

Figure 6B shows in its upper register depth-dose profiles (normalised to entrance dose) for the ⁴He ion beam, plotted as normalized dose *D_{N}* against depth in phantom d (mm); the solid curve indicates dose, while the dashed curve indicated predicted biological dose. The "x" at (140, 0) indicates the proximal face of the NCI region.

Figure 6B shows in its lower register the energy spectrum used to generate the ⁴He ion beam, plotted as normalized energy weight *W_{N}* against beam energy E (MeV/u).

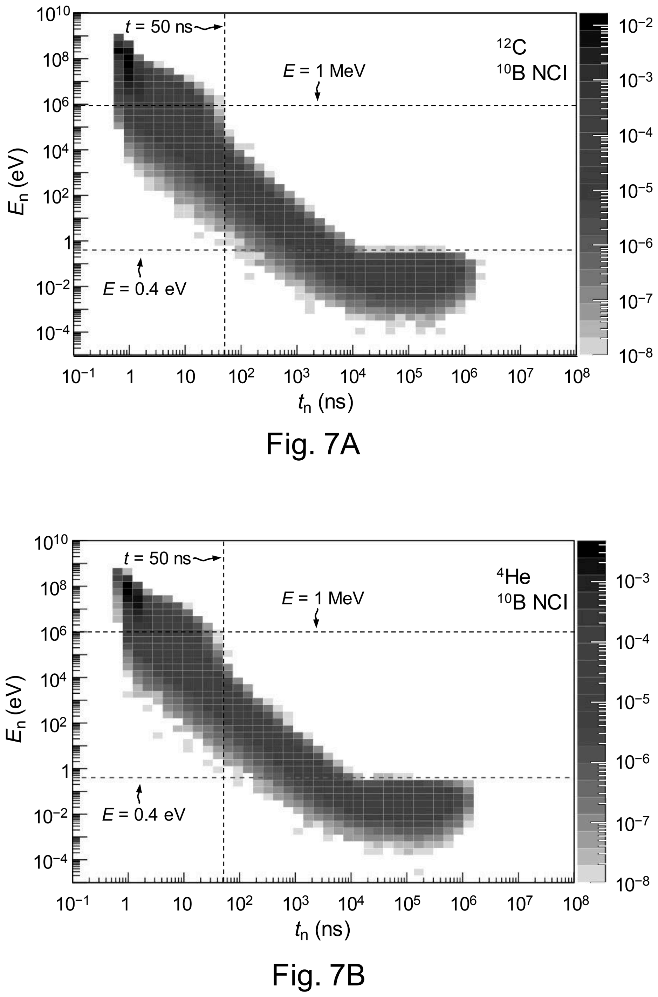

Figures 7A and 7B present two-dimensional spectrograms of neutron arrival time *t*ₙ (ns) in the NCI region (in these examples, of ¹⁰B), following irradiation by polyenergetic ¹²C and ⁴He ion beams, respectively. The spectrograms are plots of neutron energy *E*ₙ (eV) versus neutron arrival time *t*ₙ (ns). The density of each data point is indicative of the number of neutrons per primary particle, calibrated as shown by the scale to the right of each spectrogram.

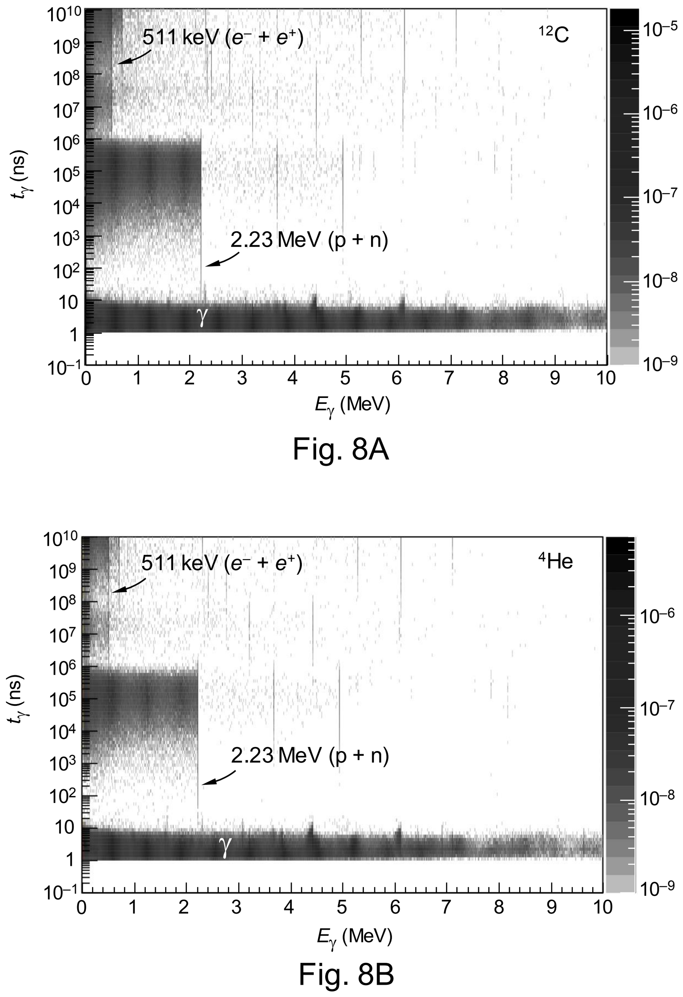

Figures 8A to 8F are spectrograms of photon detector volume arrival time *t*_{γ} (ns) as a function of gamma-ray energy *E*_{γ} (MeV) for, respectively, a ¹²C ion beam with no NCI, a ⁴He ion beam with no NCI, a ¹²C ion beam with a ¹⁰B NCI, a ⁴He ion beam with a ¹⁰B NCI, a ¹²C ion beam with a ¹⁵⁷Gd NCI, and a ⁴He ion beam with a ¹⁵⁷Gd NCI. (All ion beams were polyenergetic.) The density of each data point is indicative of the number of photons per primary particle, calibrated as shown by the scale to the right of each spectrogram.

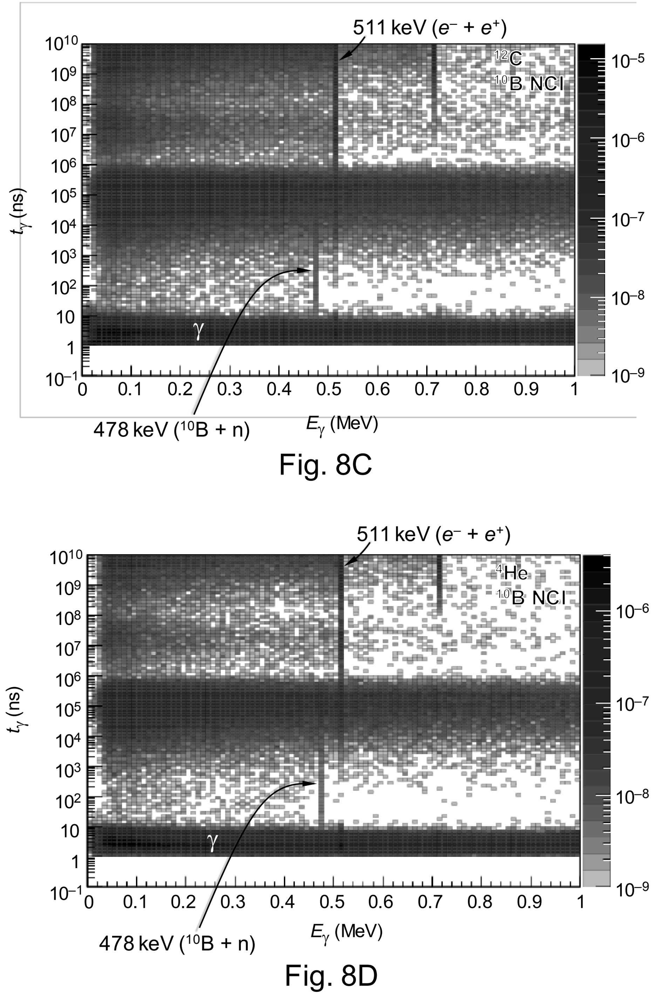

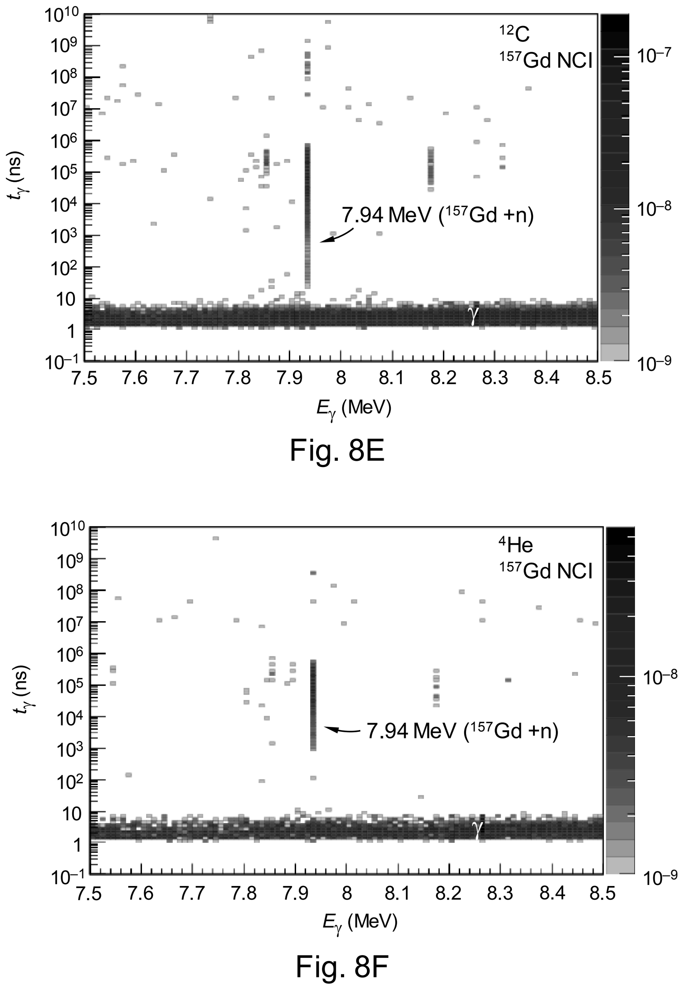

The band of gamma-rays (labelled 'y') extending across the lower portion of each of Figures 8A to 8F (arriving between 1 and 10-12 ns) corresponds to prompt (non-neutron-capture) photons. Delayed 511 keV annihilation photons are indicated (cf. Figures 8A to 8D), as are 2.23 MeV photons from hydrogen neutron capture (cf. Figures 8A and 8B), 478 keV photons from boron neutron capture (cf. Figures 8C and 8D), and 7.94 MeV photons from gadolinium neutron capture (cf. Figures 8E and 8F).

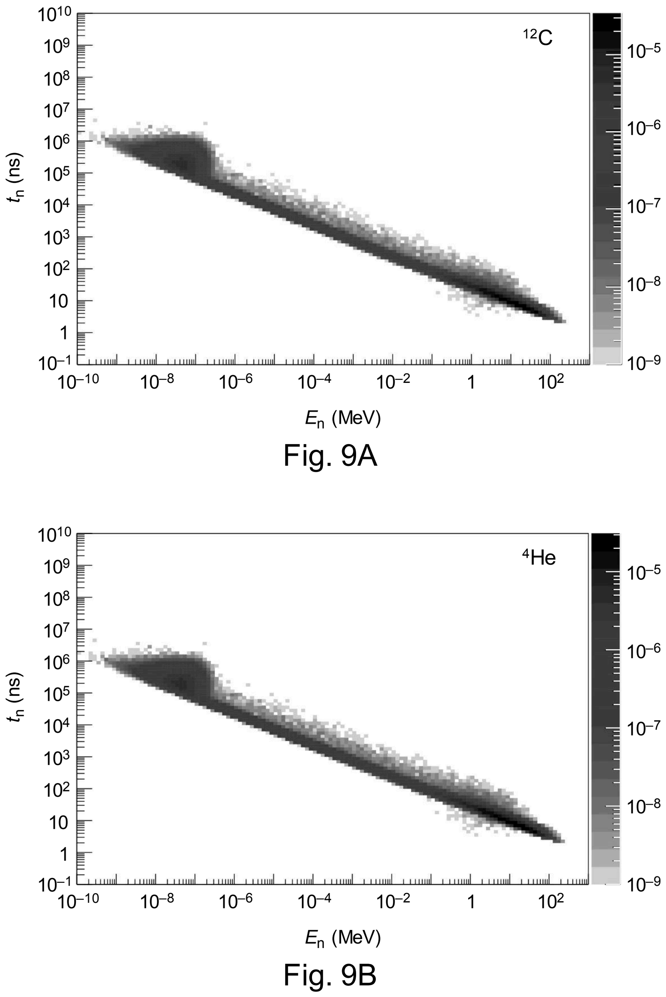

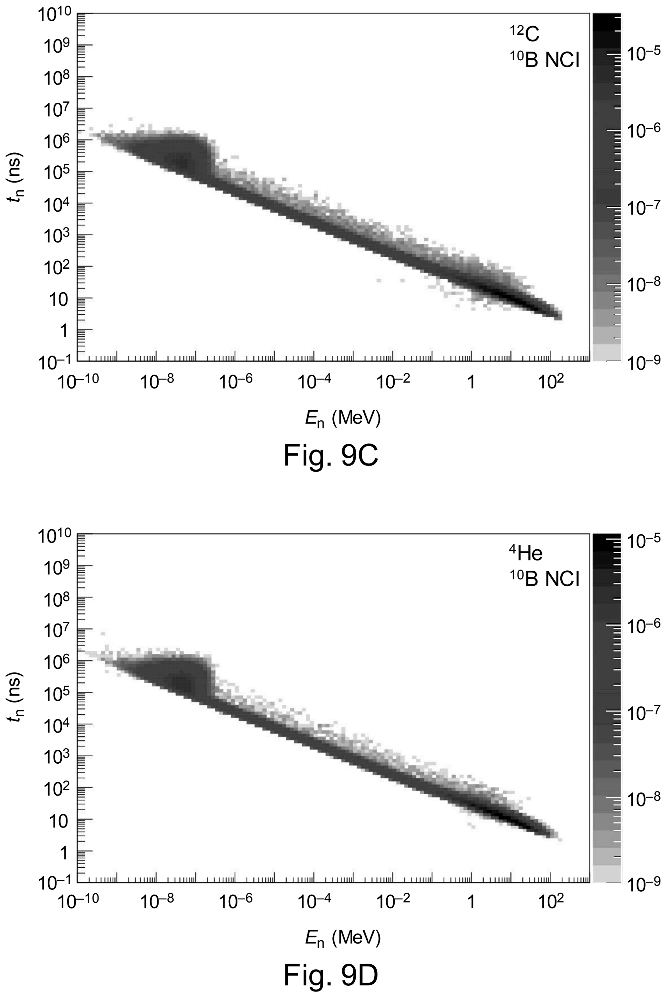

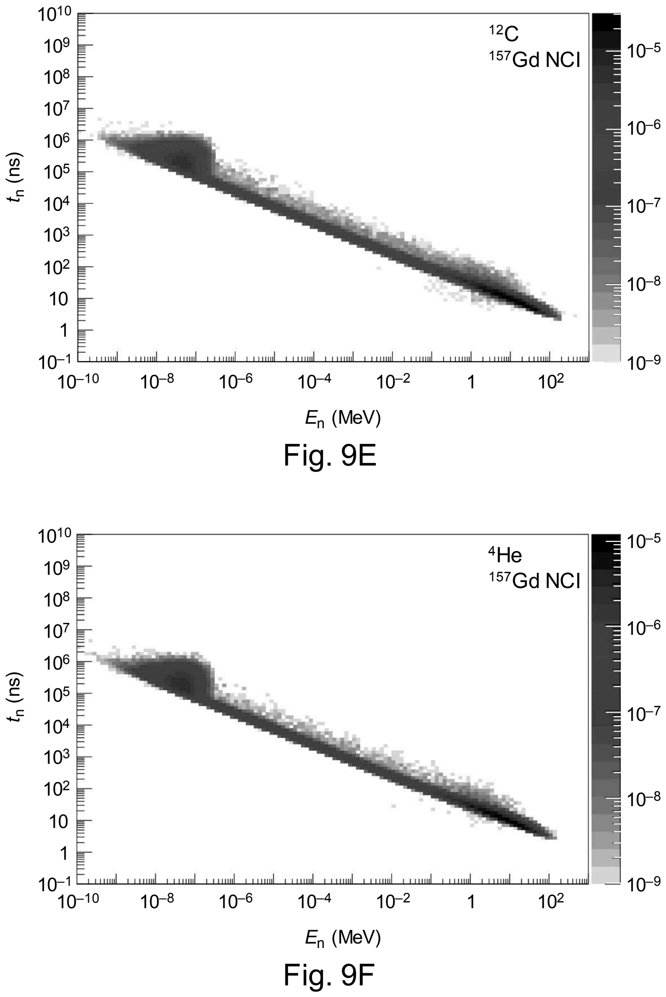

Figures 9A to 9F are spectrograms of neutron detector volume arrival time *t*ₙ (ns) as a function of the neutron energy *E*ₙ (MeV) for, respectively, a ¹²C ion beam with no NCI, a ⁴He ion beam with no NCI, a ¹²C ion beam with a ¹⁰B NCI, a ⁴He ion beam with a ¹⁰B NCI, a ¹²C ion beam with a ¹⁵⁷Gd NCI, and a ⁴He ion beam with a ¹⁵⁷Gd NCI. (All ion beams were polyenergetic.) The density of each data point is indicative of the number of neutrons per primary particle, calibrated as shown by the scale to the right of each spectrogram.

### Detector material, irradiation duration and shield optimisation

A search was undertaken for the best temporal mask and detector. For a carbon ion beam, it was determined that the CdTe detector with a 1 *µ*s irradiation period (i.e. beam duration) was the optimal combination for the boron neutron capture insert, since it provided the highest *true positives: false positives* ratio (*R_{TF}*), both for the unshielded detector and a detector shielded with 1 mm of cadmium. For the gadolinium neutron capture insert, the LSO:Ce detector with a 1 ms beam duration was the optimal combination, again providing the highest values of *R_{TF}* for both the unshielded detector and a detector shielded with 1 mm of boron.

For a helium ion beam, the optimal beam duration was determined to be the same as that for a carbon ion beam and with a boron neutron capture insert (viz. 1 *µ*s) but, with a gadolinium neutron capture insert, the optimal beam duration was determined to be 10 *µ*s rather than 1 ms.

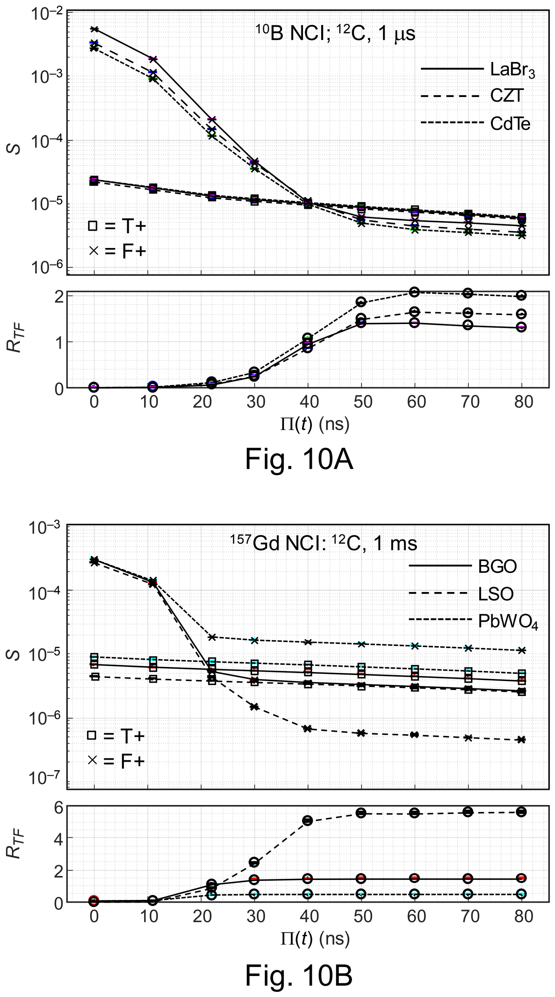

A selection of results for carbon ion irradiation are presented in Figures 10A to 13B, each with an upper register that plots sensitivity S (normalized to the number of photons incident on the detector) and a lower register that plots *R_{TF}.* In the upper registers, square markers (□)denote true positives (T+), while cross markers (×) denote false positives (F+).

Figures 10A and 10B present plots comparing the different detector material sensitivity S (upper registers) and *R_{TF}* (lower registers), for ¹⁰B and ¹⁵⁷Gd NCIs, respectively, as functions of the duration of the temporal mask Π(*t*) (ns), for realistic detector models following irradiation by a ¹²C ion beam and for all detected events. The total irradiation period was 1 *µ*s (Figure 10A) and 100 ms (Figure 10B).

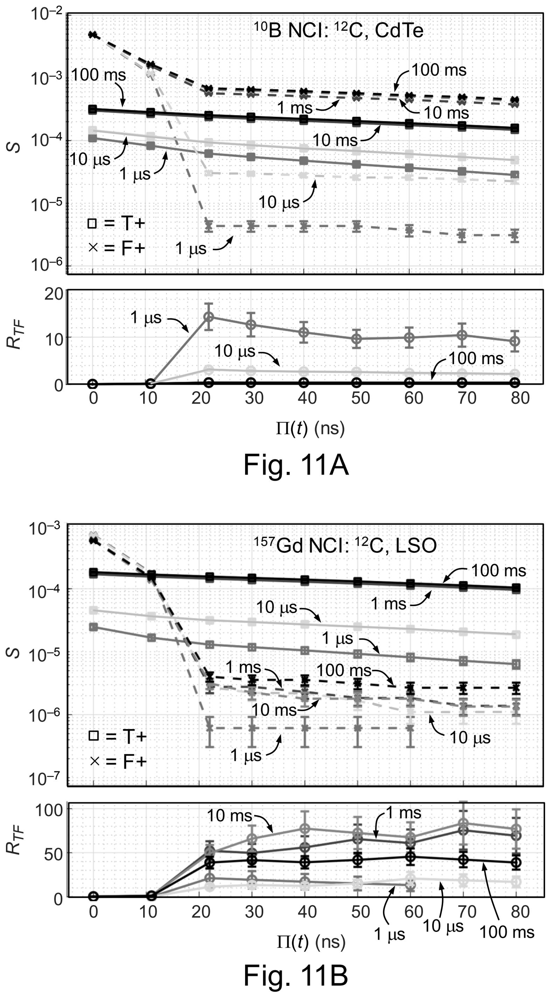

Figures 11A and 11B present plots of sensitivity S (upper registers) and *R_{TF}* (lower registers) as a function of temporal mask duration for the two best detector materials for ¹⁰B and ¹⁵⁷Gd NCIs, respectively, following irradiation by a ¹²C ion beam for a range of total irradiation periods (from 1 *µ*s to 100 ms, as labelled), for photons only-that is, excluding other particles interacting with the detector (which can be done in a simulation).

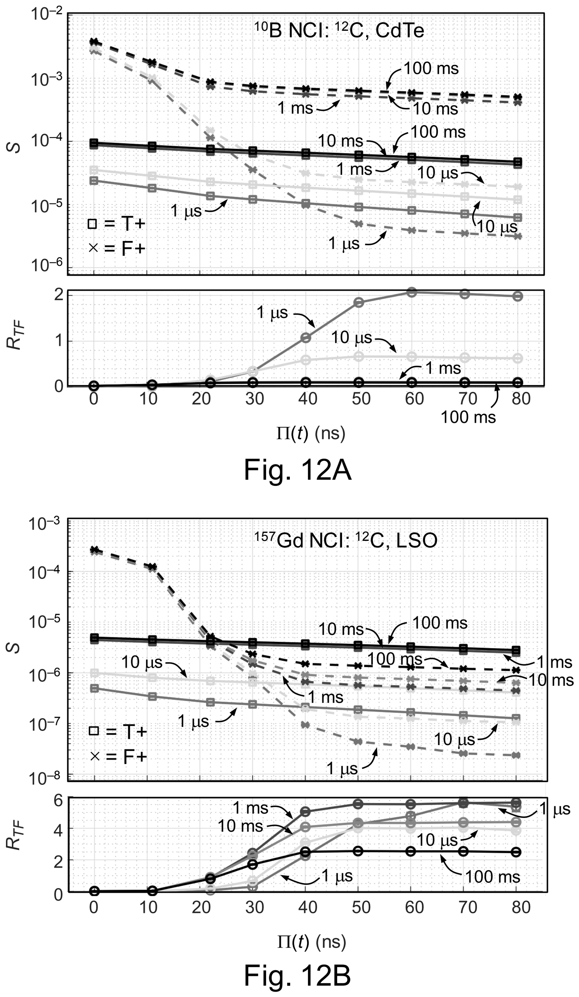

Figures 12A and 12B present plots of sensitivity S (upper registers) and *R_{TF}* (lower registers) as a function of the duration of the temporal mask Π(*t*) (ns) for the two best detector materials for ¹⁰B and ¹⁵⁷Gd NCIs, respectively, following target irradiation by a ¹²C ion beam for a range of total irradiation periods (from 1 *µ*s to 100 ms, as labelled) and for all detected events. Note that, in the lower register of Figure 12A, the curves for 1 ms and 100 ms largely coincide.

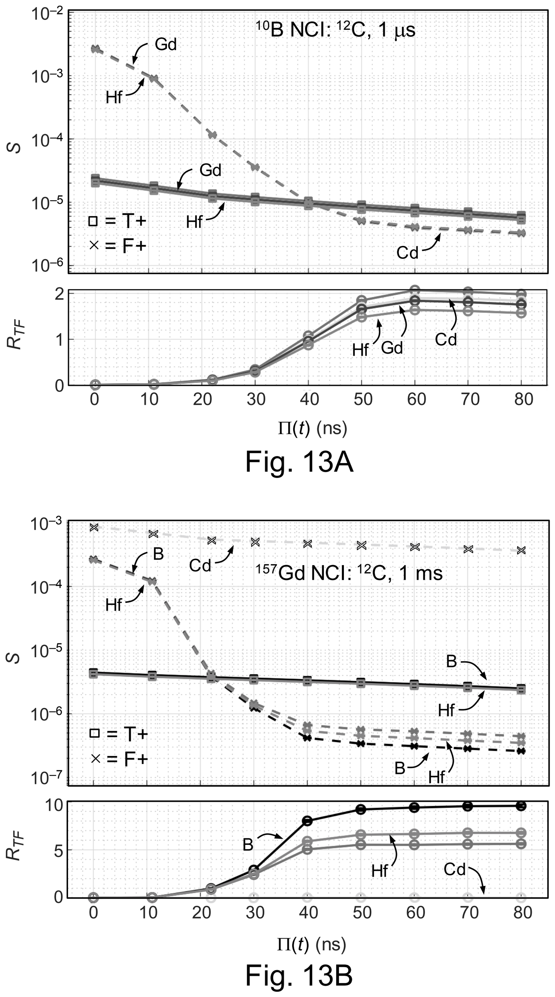

Figures 13A and 13B present plots of S (upper registers) and *R_{TF}* (lower registers) as a function of the duration of the temporal mask Δ*t* (ns), for events recorded in realistic detectors using both no front shielding and various front shielding materials (viz. Cd, Gd, B and Hf) with high thermal neutron cross-section, for ¹⁰B and ¹⁵⁷Gd NCIs respectively, following target irradiation by a carbon ion beam. The total irradiation period was 1 *µ*s (Figure 13A) and 100 ms (Figure 13B).

In the upper register of Figure 13A, the cadmium-shielded detector curve for false positives is generally obscured by the gadolinium- and hafnium-shielded detector curves. The unlabelled curves correspond to an unshielded detector. In the upper register of Figure 13A, this curve for true positives lies immediately above the gadolinium-shielded detector curve; in the upper register of Figure 13B, this curve is obscured by the boron-shielded detector curve.

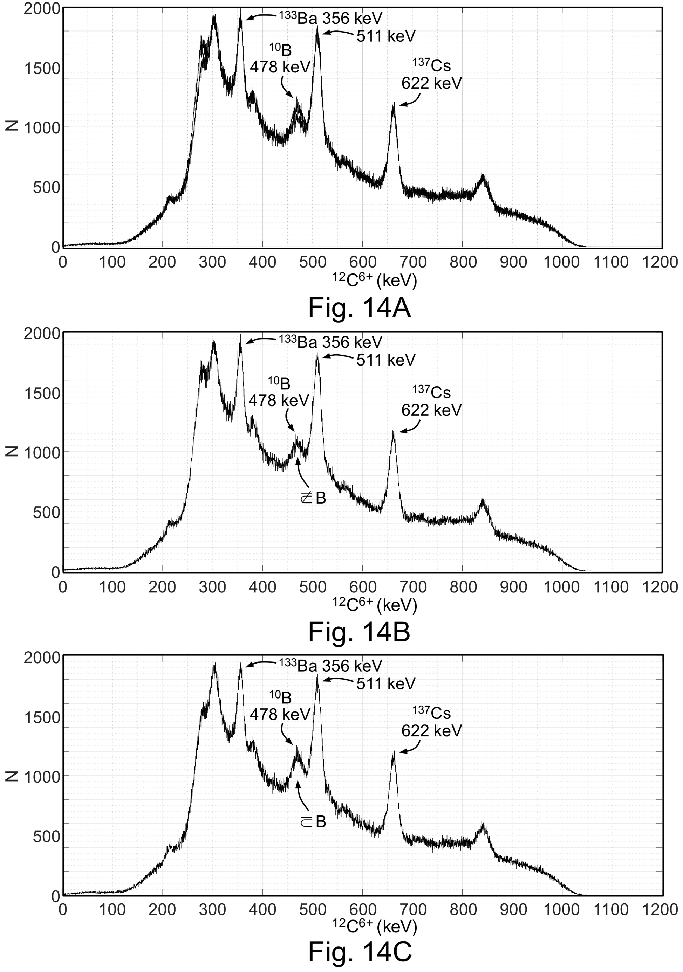

It has also been found that the gamma-ray energy signature may be strong enough for the detection of a change in the magnitude of the 478 keV gamma-ray line arising from ¹⁰B thermal neutron capture. This indicates that the thermal neutron flux that is generated internally (i.e. within the phantom or subject) is greater than predicted in simulations. Figures 14A is a plot of the number of counts (N) versus ¹²C ion beam energy (keV); this figure plots empirical data collected without a ¹⁰B NCI (Figure 14B) superimposed on empirical data collected with a ¹⁰B NCI (see Figure 14C), under the same experimental conditions. The NCI comprised five ¹⁰B plates.

It will be noted that the two datasets generally differ little over the beam's energy range, but that the 478 keV gamma-ray line is clearly greater with the NCI than without.

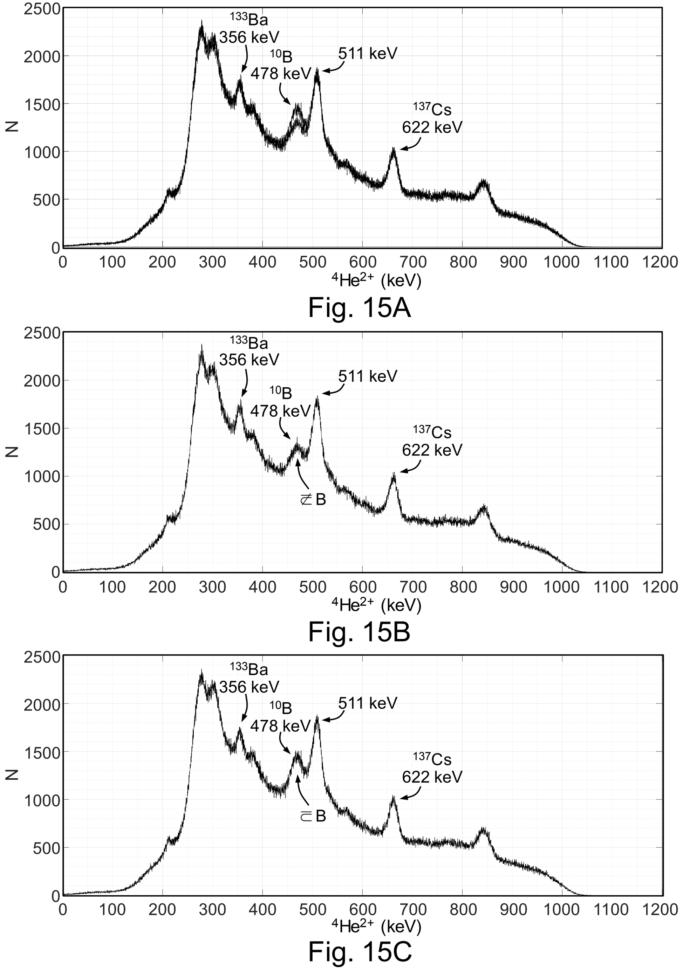

Comparable results were obtained with a ⁴He ion beam. Figures 15A is a plot of the number of counts (N) versus ⁴He ion beam energy (keV); this figure also plots empirical data collected without a ¹⁰B NCI (Figure 15B) superimposed on empirical data collected with a ¹⁰B NCI (see Figure 15C), under the same experimental conditions. The NCI comprised five ¹⁰B plates and a ¹⁰B cube. The two datasets again generally differ little over the beam's energy range, but the 478 keV gamma-ray line is clearly greater with the NCI than without.

### Discussion

From the energy and timing distribution of the neutrons entering the NCI region (cf. Figures 7A and 7B), it is apparent that arrival time of the neutrons depends on the energy of the neutrons. Neutrons that are created with high initial kinetic energies must scatter multiple times to reach thermal equilibrium, which takes time. The neutron spectrograms for both carbon and helium ion beams exhibit similar characteristics, with thermal neutrons reaching the NCI region between ~ 12 ns and 10⁶ ns after creation. As a result of the delay between primary particle arrival and thermal neutron arrival time in the NCI, it is expected that gamma emissions due to the thermal neutron capture process within the NCI will start to be observed between 12 ns and 10⁶ ns after the onset of a beam pulse.

In phantoms without an NCI (cf. Figures 8A and 8B for carbon and helium ion beams, respectively), all prompt (non-neutron-capture) photons arrived at the detector during the first 11 ns after primary particle generation. As discussed above, thermalisation takes time, so thermal neutrons appear principally during the 12 ns to 10⁶ ns window (as can be seen from the 2.23 MeV hydrogen neutron capture line), with almost none appearing outside that window. Hence, the use of a 12 ns to 10⁶ ns timing window for thermal neutron capture discrimination is justified. In the phantoms both with and without an NCI, the 511 keV annihilation line starts to appear at approximately 10⁴ ns and grows in intensity as time increases and the positron-emitting fragmentation products decay (noting that binning is logarithmic in the time direction).

In the phantoms with a ¹⁰B NCI (cf. Figures 8C and 8D for carbon and helium ion beams, respectively), the 478 keV line from ¹⁰B thermal neutron capture is visible during the period extending from approximately 12 ns to 10⁶ ns, corresponding to the period when thermal neutrons are present in the phantom. However, the presence of the strong 511 keV positron annihilation signal nearby is a confounding factor in neutron capture discrimination, demanding high energy resolution from candidate detectors. The 2.23 MeV hydrogen neutron capture contributes to background at a broad range of lower energies, due to Compton scattering.

In the case of phantoms with a ¹⁵⁷Gd NCI (cf. Figures 8E and 8F for carbon and helium ion beams, respectively), the 7.94 MeV photon line from ¹⁵⁷Gd neutron capture is visible within the same time window as the ¹⁰B NCI line (viz. ~12 ns to 10⁶ ns), predominantly from 12 ns to 10⁶ ns with a carbon ion beam and predominantly from 750 ns to 10^{5.8} ns for a helium ion beam. However, unlike in the ¹⁰B case, there is no significant scatter background, owing to the absence of nearby decay or capture peaks.

In all of the phantoms, the time of arrival of neutrons at the detector depended on neutron energy (cf. Figures 9A to 9F). This study consider three energy and timing bands of neutrons arriving at the detector, defined for present purposes as follows:
- Fast neutrons: energies above 1 MeV, with most neutrons arriving before 50 ns;
- Intermediate-energy neutrons: energies between 0.4 eV and 1 MeV, with most neutrons arriving between 50 ns and 10⁴ ns; and
- Thermal neutrons: energies below 0.4 eV, with most neutrons (> 99.9%) arriving between 10⁴ and 2 × 10⁶ ns. (Thermal neutrons are generally defined, based on Boltzmann temperature, to be those with a mean kinetic energy of around 0.025 eV; neutron energies below 0.4 eV embrace what are more commonly termed cold neutrons, thermal neutrons and epithermal neutrons. However, as will be appreciated by the skilled person, in therapeutic neutron capture applications the cadmium edge of around 0.5 eV is accepted as a more relevant threshold, which has informed the choice of 0.4 eV as the upper bound of this energy band.)

As discussed above, fast and intermediate energy neutrons can potentially deposit enough energy in the detector to satisfy the energy window for neutron-capture prompt gamma detection, thereby creating false positives. Shielding against these neutrons is impractical, since stopping energetic neutrons at these requires large amounts of shielding material, which would also attenuate the gamma photons emitted during neutron capture processes in the target. The detection of fast neutrons can be greatly reduced by using a 50 ns temporal mask post irradiation, since any fast neutrons arriving at the detector will do so within approximately 50 ns of the end of the beam pulse (providing that the detector itself does not become activated with long-lived radio-isotopes). Intermediate energy neutrons arrive at the detector in lower fluences and over a longer timescale compared to fast and thermal neutrons; while they potentially can deposit enough energy to trigger a false positive, they are much fewer in number compared to fast neutrons, and in any case, cannot be effectively blocked by shielding without compromising detector sensitivity.

For all ideal photon detectors, when the target includes a ¹⁰B NCI (cf. Figure 11A), *R_{TF}* increases as the temporal mask is widened up to 22 ns, after which the ratio begins to fall. This is because a 22 ns temporal mask is sufficient to suppress detection of non-neutron-capture prompt gamma emission, since they are only produced up to 11 ns post (end of) beam injection. *R_{TF}* decreases with increased beam duration, due to the resulting increase in photon fluence at the higher-energy emission peaks, since these photons can Compton-scatter and potentially deposit energy in the detector within the 478 keV energy window (cf. Figure 8A). In the case of a ¹⁵⁷Gd NCI (cf. Figure 11B), *R_{TF}* increases as the temporal mask is widened, reaching a maximum value with a 40 ns mask interval. However, because the absolute number of true and false positive counts is very low (i.e. the sensitivity is poor) with this mask, the 22 ns temporal mask produces the best overall balance between selectivity and sensitivity for ideal photon detectors. Furthermore, *R_{TF}* increases with increased beam duration, reaching a maximum value when the beam duration is 1 ms. This is due to the absence of high-energy emission peaks above 7.94 MeV. By increasing the beam duration, and hence acquisition time, a higher proportion of photons from the neutron capture insert (true positives) can be detected without increasing the false positive rate.

For all evaluated physical detector materials, and for both ¹⁰B and ¹⁵⁷Gd neutron capture inserts, *R_{TF}* increases to its maximum value as the temporal mask duration is increased to ~ 60 ns, after which the ratio starts to plateau (cf. Figures 12A and 12B). Fast neutrons cannot be effectively shielded, and any which deposit energy in the detector will do so within the first 60 ns, increasing the false negative count and decreasing *R_{TF}.* For phantoms with a ¹⁰B neutron capture insert, *R_{TF}* falls with increasing beam duration owing to the increasing prevalence of scattered photons with initial energies above the neutron capture photopeak. For the ¹⁵⁷Gd NCI, *R_{TF}* continues to increase as irradiation time increases owing to both a lack of higher energy photons, and the small number of fast and intermediate-energy neutrons capable of depositing energy in the energy acceptance window.

For a carbon ion beam with a 60 ns temporal mask (cf. Figure 10A), the detector material that achieved the highest *R_{TF}* with a ¹⁰B NCI was CdTe (*R_{TF} =* 2.07 ± 0.01) followed by CZT (*R_{TF} =* 1.645 ± 0.009) and finally LaBr₃ (*R_{TF} =* 1.402 ± 0.007).

For a carbon ion beam and a 60 ns temporal mask (cf. Figure 10B), the detector material that achieved the highest *R_{TF}* with the ¹⁵⁷Gd NCI was LSO (*R_{TF} =* 5.52 ± 0.06), followed by BGO (*R_{TF} =* 1.454 ± 0.008) and finally PbWO₄ (*R_{TF} =* 0.442 ± 0.002). Unlike the ¹⁰B NCI case, there is an absence of scatter from high energy photons, which implies that all false positives will be a result of neutron interactions within the detector. The PbWO₄ detector had the highest absolute sensitivity to true positives but, for all temporal masks, the false positive rates were higher than the true positive rates. This suggests that if the detector could be sufficiently shielded against intermediate energy neutrons to reduce the rate of false positives, it might be a more competitive choice of detector material. Additionally, the detector with the highest *R_{TF}* was the LSO detector despite having the lowest sensitivity to true positives, as neutrons caused the lowest rate of false positives compared to the other detectors.

**Thermal** neutrons start to arrive at the detector in significant numbers after 10⁴ ns. Owing to their low kinetic energy, thermal neutrons do not directly contribute to false positives; however, they can cause the detector itself to become activated, contributing to an increase in background radiation, which, in turn, can result in the detection of false positives (depending on the wavelengths of emitted gamma radiation). As the detector ages, this problem is expected to progressively increase, so it is desirable to block thermal neutrons from the detector.

The addition of a thin layer of any of the evaluated shielding materials on the front face of the detector results in a very small decrease in *R_{TF}* when using phantoms with a ¹⁰B neutron capture insert (cf. Figure 13A). This is a result of slightly increased attenuation and scattering of the neutron capture gamma photons prior to reaching the sensitive volume of the detector, while there is minimal impact on the background level of scattered photons, fast and intermediate neutrons.

In the case of the ¹⁵⁷Gd neutron capture insert, the addition of hafnium or boron front shielding on the detector resulted in an increase in *R_{TF},* with hafnium (*R_{TF}* = 6.65 ± 0.08) and boron (*R_{TF} =* 9.3 ± 0.1), as false positives are predominantly caused by neutron interactions within the detector (cf. Figure 13B). However, the addition of cadmium shielding with a ¹⁵⁷Gd NCI in the target results in a very substantial decrease in *R_{TF}* (*R_{TF}* = 0.00667 ± 0.00003), since the ¹¹³Cd neutron capture reaction results in the emission of high energy photons at 8.48 MeV and 9.04 MeV, which will cause single and double escape peaks to fall in the 7.94 MeV energy acceptance band [Ref. 31].

The effect of thermal neutron shielding almost entirely eliminates the problem of thermal neutron activation of the detector while having minimal negative effect on the sensitivity and specificity of the prompt gamma detector system (with the exception of cadmium shielding in the case of a ¹⁵⁷Gd-bearing target), so the use of thermal neutron shielding to prolong the life of the detector is justified.

Finally, it will be noted that these simulations (and the preceding discussion) focus on carbon ion and helium ion beams. The simulations also support, however, the use of dose quantification system 20 with other beam types, subject only to fine tuning the timing windows (whether by simple trials or simulation). This is because system 20 relies on principles common to all beam types: the detection of gamma-rays of known energies from thermal neutron capture, and the rejection of detection events due to prompt gamma-rays and thermal neutrons.

Similarly, these simulations (and the preceding discussion) focus on neutron capture agents in the form of ¹⁰B and/or ¹⁵⁷Gd. This is because they are currently the best agents in terms of getting them to the tumour or lesion site without harming the subject. However, system 20 would be suitable for other neutron capture agents, as system 20 employs an energy window that is adjustable to correspond to the energy (or energies) of the neutron capture gamma-rays in each case. Likewise, it is a straightforward matter to select the type of gamma-ray detector 22 according to the gamma-rays to be detected, and to select shielding material (if employed) so as to be distinct from the neutron capture agent.

Furthermore, the examples and simulations presented above are based on a beam-on period of 11 ns, as this is a beam-on period delivered by a number of synchrotrons. This period may differ with other synchrotrons or beam delivery apparatuses, but the duration of temporal mask Δ*t* (ns) need merely be adjusted accordingly to preserve the temporal extent of the mask after the end of each beam-on period.

In addition, while the simulations identified variations combinations of parameters that optimized *R_{TF},* acceptable if not optimal dose or dose distribution results could be obtained with *R_{TF}* values of as low as 1.4 or 1.5.

### Conclusion

These simulations demonstrate the feasibility of the method for discriminating between ¹⁰B or ¹⁵⁷Gd neutron capture events and other sources of prompt gamma radiation from the target volume during particle therapy, via energy windowing and temporal masking relative to the beam pulse arrival time.

Overall, for targets containing a ¹⁰B NCI, the detector that obtained the highest *R_{TF}* was the CdTe detector with a 60 ns temporal mask and 1 *µ*s irradiation duration. Acceptable values of *R_{TF}* were, however, obtained with shorter temporal masks (e.g. as low as 50 ns).

For the ¹⁵⁷Gd NCI, the LSO detector provided the highest *R_{TF}* with a 60 ns temporal mask and 1 ms beam duration (though acceptable values of *R_{TF}* were obtained with shorter temporal masks (e.g. as low as 50 ns, or 40 ns with a carbon ion beam). The addition of a thin thermal-neutron shield to the front face of the detector results in a slight reduction in sensitivity and selectivity when using a boron NCI, but it allows almost all of the thermal neutrons to be absorbed before reaching the detector, avoiding the problem of neutron activation. In the case of the gadolinium NCI, the addition of front face shielding results in an increase in the *R_{TF},* as the false positives are caused by neutron interactions within the detector.

Modifications within the scope of the invention may be readily effected by those skilled in the art. It is to be understood, therefore, that this invention is not limited to the particular embodiments described by way of example hereinabove.

In the claims that follow and in the preceding description of the invention, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention.

Further, any reference herein to prior art is not intended to imply that such prior art forms or formed a part of the common general knowledge in any country.

### References

[1] M. Safavi-Naeini et al. Opportunistic dose amplification for proton and carbon ion therapy via capture of internally generated thermal neutrons. Sci. Reports 8 (2018). URL https://doi.org/10.1038%2Fs41598-018-34643-w. DOI 10.1038/s41598-018-34643-w.
[2] Sauerwein, W., Wittig, A., Moss, R. & Nakagawa, Y. (eds.). Neutron Capture Therapy, 164-165 (Springer Berlin Heidelberg, 2012).
[3] Goorley, T. & Nikjoo, H. Electron and photon spectra for three gadolinium-based cancer therapy approaches. Radiat. Res. 154, 556-563 (2000). DOI 10.1667/0033-7587(2000)154[0556:eapsft]2.0.co;2.
[4] Tanaka, T. et al. Gamma-ray spectra from thermal neutron capture on gadolinium-155 and natural gadolinium. Prog. Theor. Exp. Phys. 2020(4) (2020) 043D02. DOI 10.1093/ptep/ptaa015.
[5] Dymova, M. A., Taskaev, S. Y., Richter, V. A. & Kuligina, E. V. Boron neutron capture therapy: Current status and future perspectives. Cancer Commun. 40 (2020) 406-421. DOI 10.1002/cac2.12089.
[6] Sakurai, Y. & Kobayashi, T. Experimental verification of the nuclear data of gadolinium for neutron capture therapy. J. Nucl. Sci. Technol. 39 (2002) 1294-1297. DOI 10.1080/00223131.2002.10875341.
[7] Kelleter, L. et al. Spectroscopic study of prompt-gamma emission for range verification in proton therapy. Phys. Medica 34 (2017) 7-17. DOI 10.1016/j.ejmp.2017.01.003.
[8] Foulher, F. L. et al. Monte carlo simulations of prompt-gamma emission during carbon ion irradiation. IEEE Transactions on Nucl. Sci. 57 (2010) 2768-2772. DOI 10.1109/tns.2010.2048042.
[9] Testa, E. et al. Dose profile monitoring with carbon ions by means of prompt-gamma measurements. Nucl. Instruments Methods Phys. Res. Sect. B: Beam Interactions with Mater. Atoms 267 (2009) 993-996. DOI 10.1016/j.nimb.2009.02.031.
[10] Min, C.-H., Kim, C. H., Youn, M.-Y. & Kim, J.-W. Prompt gamma measurements for locating the dose falloff region in the proton therapy. Appl. Phys. Lett. 89 (2006) 183517. DOI 10.1063/1.2378561.
[11] Richter, C. et al. First clinical application of a prompt gamma based in vivo proton range verification system. Radiother. Oncol. 118 (2016) 232-237. DOI 10.1016/j.radonc.2016.01.004.
[12] Parodi, K. & Polf, J. C. In vivo range verification in particle therapy. Med. Phys. 45 (2018). DOI 10.1002/mp.12960.
[13] Bello, R. D., Martins, P. M. & Seco, J. CeBr3 scintillators for 4He prompt gamma spectroscopy: Results from a monte carlo optimization study. Med. Phys. 45, 1622-1630 (2018). DOI 10.1002/mp.12795.
[14] Zarifi, M. et al. Characterization of prompt gamma ray emission for in vivo range verification in particle therapy: A simulation study. Phys. Medica 62 (2019) 20-32. DOI 10.1016/j.ejmp.2019.04.023.
[15] Testa, M. et al. Real-time monitoring of the bragg-peak position in ion therapy by means of single photon detection. Radiat. Environ. Biophys. 49 (2010) 337-343. DOI 10.1007/s00411-010-0276-2.
[16] Hueso-González, F. et al. Compton camera and prompt gamma ray timing: Two methods for in vivo range assessment in proton therapy. Front. Oncol. 6 (2016). DOI 10.3389/fonc.2016.00080.
[17] Werner, T. et al. Processing of prompt gamma-ray timing data for proton range measurements at a clinical beam delivery. Phys. Medicine & Biol. 64 (2019) 105023. DOI 10.1088/1361-6560/ab176d.
[18] Agostinelli, S. et al. Geant4a simulation toolkit. Nucl. Instruments Methods Phys. Res. Sect. A: Accel. Spectrometers, Detect. Assoc. Equip. 506 (2003) 250-303. DOI 10.1016/s0168-9002(03)01368-8.
[19] Bolst, D. et al. Validation of geant4 fragmentation for heavy ion therapy. Phys. Medica 42 (2017) 4. DOI 10.1016/j.ejmp.2017.09.010.
[20] Chacon, A. et al. Comparative study of alternative geant4 hadronic ion inelastic physics models for prediction of positron-emitting radionuclide production in carbon and oxygen ion therapy. Phys. Medicine & Biol. 64 (2019) 155014. DOI 10.1088/1361-6560/ab2752.
[21] Geant4 Collaboration. Geant4 material database. http://geant4-userdoc.web.cern.ch/geant4-userdoc/UsersGuides/ForApplicationDeveloper/html/Appendix/materialNames.html.
[22] Advatech. LSO(Ce) - Lutetium Oxyorthosciilicate (Ce) Scintillator Crystal. Available URL: https://www.advatech-uk.co.uk/lso_ce.html (2021).
[23] Crystals, S.-G. BGO Bismuth Germanate Bi4Ge3O12. Available URL: https://www.crystals.saint-gobain.com/products/bgo (2021).
[24] Crystals, S.-G. Lanthinum Bromide LaBr3(Ce). Available URL: https://www.crystals.saint-gobain.com/products/standard-and-enhanced-lanthanum-bromide (2021).
[25] Advatech. LaBr3(Ce) - Scintillator Crystal. Available URL: https://www.advatech-uk.co.uk/labr3_ce.html (2021).
[26] MSE Supplies LLC. Lead-Tungstate Crystals PbWO4. Available URL: https://www.msesupplies.com/products/pbwo4-crystals-lead-tungstate (2021).
[27] Danevich, F. et al. Application of PbWO4 crystal scintillators in experiment to search for decay of 116cd. Nucl. Instruments Methods Phys. Res. Sect. A: Accel. Spectrometers, Detect. Assoc. Equip. 556 (2006) 259-265. DOI 10.1016/j.nima.2005.09.049.
[28] Murata, I., Nakamura, S., Manabe, M., Miyamaru, H. & Kato, I. Characterization measurement of a thick CdTe detector for BNCT-SPECT detection efficiency and energy resolution. Appl. Radiat. Isot. 88 (2014) 129-133. DOI 10.1016/j.apradiso.2014.01.023.
[29] Yamada, S. Commissioning and performance of the HIMAC medical accelerator. In Proceedings Particle Accelerator Conference (IEEE). DOI 10.1109/pac.1995.504557.
[30] Fujimoto, T. et al. Acceleration of heavy ions with a new RF system at HIMAC synchrotron. Nucl. Instruments Methods Phys. Res. Sect. B: Beam Interactions with Matter. Atoms 269 (2011) 2886-2890. DOI 10.1016/j.nimb.2011.04.029.
[31] Rusev, G. et al. Cascade gamma rays following capture of thermal neutrons on 113cd. Phys. Rev. C 88 (2013). DOI 10.1103/physrevc.88.057602.

## Claims

1. A radiation dose quantification method, comprising:
detecting, with one or more detectors (22) with respective sensitive volumes (24), gamma-rays emitted as a result of capture of neutrons (32) by a composition in a subject (10) subjected to an irradiation program, the composition comprising one or more thermal neutron capture agents, the neutrons having been generated by non-elastic collisions between a primary beam (16) of charged particles and nuclei in the subject (10), wherein the charged particles consist of any one or more of protons, deuterons, tritons and heavy ions, the irradiation program comprises at least one period of irradiation with a beam duration that includes beam-on periods and beam-off periods;
applying at least one predefined energy window (*E*_{L} *- E*_{H}) or filter configured to accept only detection events in the one or more detectors (22) resulting from gamma-rays with an energy indicative of selected gamma-rays arising from the capture of thermal neutrons by the one or more thermal neutron capture agents, wherein the thermal neutrons are neutrons with energies below approximately 0.4 eV;
applying a timing window configured to reject or ignore detection events in the one or more detectors (22) resulting from at least prompt gamma-rays produced in non-neutron capture events; and
determining the radiation dose of neutron radiation received by the subject (10) during the irradiation program from at least the accepted detection events or determining a dose map of the radiation received by the subject (10) from at least the accepted detection events.

2. A method as claimed in claim 1, including configuring the timing window to reject:
i) the prompt gamma-rays by rejecting gamma-rays that arrive in the respective sensitive volumes (24) from the start of each beam-on period to approximately 11 ns, approximately 12 ns, or from 10 to 12 ns, after the end of the respective beam-on period; and/or
ii) detection events in the respective sensitive volumes (24) attributable, based on timing, to fast neutrons; and/or
iii) detection events in the respective sensitive volumes (24) attributable, based on timing, to neutrons with energies between 0.4 eV and 1 MeV; and/or
iv) detection events in a detection period that has a ratio of true positive detection events to false positive detection events, ascertained empirically or by simulation, to be less than approximately 1.4 or less than approximately 1.5, wherein a true positive detection event is an event that satisfies the timing window and the energy window (*E*_{L} *- E*_{H}) and arises from a neutron capture event, and a false positive detection event is an event that satisfies the timing window and the energy window (*E*_{L} - *E*_{H}) and arises from other than a neutron capture event.

3. A method as claimed in either claim 1 or 2, comprising shielding the one or more detectors with a thermal neutron absorbing material, wherein the material is different from the one or more neutron capture agents, and optionally i) is selected to be thick enough to block most thermal neutrons but thin enough not to absorb too high a fraction of the gamma-rays due to neutron capture, and/or ii) comprises any one or more of cadmium, gadolinium, boron and hafnium.

4. A method as claimed in any one of the preceding claims, wherein the one or more neutron capture agents are ¹⁰B-based and/or ¹⁵⁷Gd-based.

5. A method as claimed in any one of the preceding claims, comprising planning a radiation therapy involving generation of thermal neutrons within a patient by beam-target nuclear interactions in and around the treatment site.

6. A method as claimed in any one of the preceding claims, wherein the neutron capture agent is ¹⁰B-based and the one or more detectors comprise any one or more of CdTe, CZT, LYSO:Ce and LaBr₃:Ce detectors and, optionally, wherein the beam duration is of from 1 to 10 *µ*s, or of approximately 1 *µ*s.

7. A method as claimed in any one of claims 1 to 5, wherein the neutron capture agent is ¹⁵⁷Gd-based and the one or more detectors comprise any one or more of LSO:Ce, BGO and PbWO₄ detectors and, optionally, wherein
i) the beam duration is of from 10 *µ*s to 100 ms; or
ii) the primary beam is a carbon ion beam and the beam duration is of approximately 1 ms; or
iii) the primary beam is a helium ion beam and the beam duration is of approximately 10 *µ*s.

8. A method as claimed in any one of claims 1 to 5, wherein:
i) the neutron capture agent is ¹⁰B-based, the one or more detectors (22) comprise any one or more of CdTe, CZT, LYSO:Ce and LaBr₃:Ce detectors, and the method comprises shielding the one or more detectors with a thermal neutron absorbing material comprising natural Cd, natural Gd and/or natural Hf; or
ii) the neutron capture agent is ¹⁵⁷Gd-based, the one or more detectors (22) comprise any one or more of LSO:Ce, BGO and PbWO₄ detectors, and the method comprises shielding the one or more detectors (22) with a thermal neutron absorbing material comprising natural B and/or natural Hf.

9. A radiation dose quantification system, comprising:
one or more detectors (22) with respective sensitive volumes (24), and configured to detect gamma-rays emitted as a result of capture of neutrons (32) by a composition in a subject (10) subjected to an irradiation program, the composition comprising one or more thermal neutron capture agents, the neutrons having been generated by non-elastic collisions between a primary beam (16) of particles and nuclei in the subject (10), wherein the particles consist of any one or more of protons, deuterons, tritons and heavy ions, the irradiation program comprises at least one period of irradiation with a beam duration that includes beam-on periods and beam-off periods;
an energy gate (46, 48) configured to apply at least one predefined energy window (*E*_{L} *- E*_{H}) or filter such that the system (20) accepts only detection events in the one or more detectors (22) resulting from gamma-rays with an energy indicative of selected gamma-rays arising from the capture of thermal neutrons by the one or more thermal neutron capture agents, wherein the thermal neutrons are neutrons with energies below approximately 0.4 eV;
a timing gate (38) configured to receive beam data indicative of start or end of the respective beam-on periods and to generate from the beam data, and apply, a timing window configured such that the system (20) rejects or ignores detection events in the one or more detectors (22) resulting from at least prompt gamma-rays produced in non-neutron capture events; and
an output for outputting data indicative of the accepted detection events.

10. A system as claimed in claim 9, wherein the one or more detectors (22) are sensitive to the angle of arrival of gamma-rays.

11. A system as claimed in either claim 9 or 10, comprising:
a data logger or data analysis device (52) configured to determine the radiation dose of the radiation received by the subject (10) during the irradiation program from the counted gamma-rays or a dose map of the radiation received by the subject (10) from the counted gamma-rays; and/or
a pileup rejector (40) configured to rejecting pileup in output signals from the one or more detectors (22); and/or
a thermal neutron shield (28) arranged to shield some or all of the one or more detectors (22) and comprising a thermal neutron absorbing material, wherein the material is different from the one or more neutron capture agents and, optionally, i) is selected to be thick enough to block most thermal neutrons, but thin enough not to absorb too high a fraction of the gamma-rays due to neutron capture; and/or ii) comprises cadmium, gadolinium, boron and/or hafnium.

12. A system as claimed in any one of claims 9 to 11, wherein the timing gate (38) is configured to apply a timing window that rejects:
i) the prompt gamma-rays by rejecting gamma-rays that arrive in the respective sensitive volumes (24) from the start of each beam-on period to ending approximately 11 ns, approximately 12 ns, or from 10 to 12 ns, after the end of the respective beam-on period; and/or
ii) detection events in the respective sensitive volumes (24) attributable, based on timing, to fast neutrons; and/or
iii) detection events in the respective sensitive volumes (24) attributable, based on timing, to neutrons with energies between 0.4 eV and 1 MeV; and/or
iv) detection events in a detection period that has a ratio of true positive detection events to false positive detection events, ascertained empirically or by simulation, to be less than approximately 1.4 or less than approximately 1.5, wherein a true positive detection event is an event that satisfies the timing window and the energy window (*E*_{L} *- E*_{H}) and arises from a neutron capture event, and a false positive detection event is an event that satisfies the timing window and the energy window (*E*_{L} - *E*_{H}) and arises from other than a neutron capture event.

13. A system as claimed in any one of claims 9 to 12, wherein the one or more neutron capture agents are ¹⁰B-based and/or ¹⁵⁷Gd-based.

14. A system as claimed in any one of claims 9 to 12, wherein the neutron capture agent is ¹⁰B-based and the one or more detectors comprise any one or more of CdTe, CZT, LYSO:Ce and LaBr₃:Ce detectors and, optionally, wherein the beam duration is of from 1 to 10 *µ*s, or of approximately 1 *µ*s.

15. A system as claimed in any one of claims 9 to 12, wherein the neutron capture agent is ¹⁵⁷Gd-based and the one or more detectors comprise any one or more of LSO:Ce, BGO and PbWO₄ detectors and, optionally, wherein
i) the beam duration is of from 10 *µ*s to 100 ms; or
ii) the primary beam is a carbon ion beam and the beam duration is of approximately 1 ms; or
iii) the primary beam is a helium ion beam and the beam duration is of approximately 10 *µ*s.

16. A system as claimed in any one of claim 9 to 12, wherein:
i) the neutron capture agent is ¹⁰B-based, the one or more detectors (22) comprise any one or more of CdTe, CZT, LYSO:Ce and LaBr₃:Ce detectors, and the system (20) comprises a thermal neutron shield (28) arranged to shield some or all of the one or more detectors (22) and of a thermal neutron absorbing material comprising natural Cd, natural Gd and/or natural Hf; or
ii) the neutron capture agent is ¹⁵⁷Gd-based, the one or more detectors (22) comprise any one or more of LSO:Ce, BGO and PbWO₄ detectors, and the system (20) comprises a thermal neutron shield (28) arranged to shield some or all of the one or more detectors (22) and of a thermal neutron absorbing material comprising natural B and/or natural Hf.
